# EUROPEAN PATENT APPLICATION

(11) **EP 1 625 855 A1**
(43) Date of publication of application: **15.02.2006**
(21) Application number: 04019293.2
(22) Date of filing: 13.08.2004
(51) Int. Cl.: A61K 47/48

(54) **Polymeric prodrug with a self-immolative linker**

(71) Applicant: Complex Biosystems GmbH, 69120 Heidelberg (DE)
(72) Inventor: Hersel, Ulrich c/o Complex Biosystems GmbH, 69120 Heidelberg (DE); Rau, Harald c/o Complex Biosystems GmbH, 69120 Heidelberg (DE); Schnepf, Robert c/o Complex Biosystems GmbH, 69120 Heidelberg (DE); Wegge, Thomas c/o Complex Biosystems GmbH, 69120 Heidelberg (DE); Vetter, Dirk c/o Complex Biosystems GmbH, 69120 Heidelberg (DE)
(74) Representative: Harrison, Robert John

(57) **Abstract**

The invention relates to a molecule having the following structure: 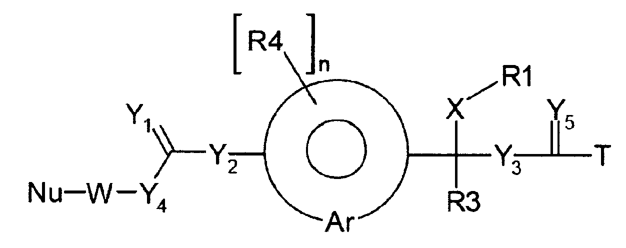 or 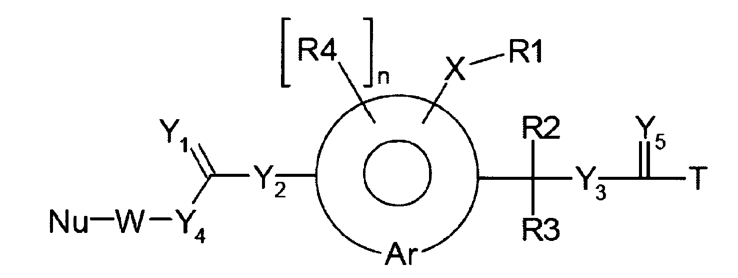 for use as a polymeric prodrug.

## Description

### Field

The present invention is directed to polymeric prodrugs having reversible linkages to amino groups of biologically active entities such as peptides, proteins, natural products or synthetic chemical compounds.

### Background

Polymers used in drug delivery systems are of interest because the therapeutic efficacy of both many low molecular weight and macromolecular medicinal agents may be significantly improved by combining them with polymers.

For example, covalent modification of biological molecules with poly(ethylene glycol) (PEG) has been extensively studied since the late 1970s. So-called PEGylated proteins have shown improved therapeutic efficacy by increasing solubility, reducing immunogenicity, and increasing circulation half-live in vivo due to reduced renal clearance and proteolysis by enzymes (see, for example, Caliceti P.,Veronese F.M., Adv. Drug Deliv. Rev. 2003, 55, 1261-1277).

Many small molecule medicinal agents, like alkaloids and anti-tumor agents, show low solubility in aqueous fluids. One way to solubilize these small molecule compounds is to conjugate them to hydrophilic polymers. A variety of water-soluble polymers, such as human serum albumin, dextran, lectins, poly(ethylene glycol), poly(styrene-co-maleic anhydride), poly(N-hydroxypropylmethacrylamide), poly(divinyl ether-co-maleic anhydride), hyaluronic acid have been described for this purpose.

Depot formulations generating long-acting release profiles have been described using non-covalent drug encapsulation. Typically, a drug is mixed with polymer material and processed in such fashion, that the drug is captured in the polymer interior.

Resulting polymer-protein aggregates may be shaped as microparticles or gels. Drug release occurs if the polymer swells or degradation of the polymer allows for diffusion of the drug to the exterior. Degradation processes may be autohydrolytic or enzyme-catalyzed. Marketed drugs based on non-covalent polymer encapsulation are Lupron Depot and Nutropin Depot. A disadvantage of this approach is that in order to prevent uncontrolled, burst-type release of the non-covalently bound drug, encapsulation has to be highly efficient, requiring strong van der Waals contacts, frequently mediated through hydrophobic moieties, and many conformationally sensitive therapeutics such as proteins or peptides are rendered dysfunctional during the encapsulation process and/or during subsequent storage. Furthermore, dependence of the release mechanism upon biodegradation may cause interpatient variability.

A major challenge in cancer therapy is to selectively target cytotoxic agents to tumor cells. A promising method to accumulate small molecule anticancer agents in tumor tissue and decrease undesirable side effects of these agents is the attachment of the cytotoxin to a macromolecular carrier. The passive targeting of polymeric drug conjugates to tumors is based on the so-called enhanced permeability and retention effect (EPR) as described by Matsumura, Y. and Maeda, H., in Cancer Res., 1986, vol 6, pp 6387-6392. As a result, several polymer-drug conjugates have entered clinical trial as anticancer agents.

However, many medicinal agents are inactive or show decreased biological activity when a polymer is covalently conjugated to the drug molecule. In these cases, a prodrug approach may be applied in such a fashion that the medicinal agent is released from the polymeric carrier in vivo to regain its biological activity. Prodrugs may be defined as therapeutic agents that are inactive per se but are predictably transformed into active metabolites (see B. Testa, J.M: Mayer in Hydrolysis in Drug and Prodrug Metabolism, Wiley-VCH, 2003, page 4). The reduced biological activity of the prodrug as compared to the released drug is of advantage if a slow or controlled release of the drug is desired. In this case, a relatively large amount of prodrug may be administered without concomitant side effects and the risk of overdosing. Release of the drug occurs over time, thereby reducing the necessity of repeated and frequent administration of the drug.

### Definitions established by IUPAC

(as given under http://www.chem.qmul.ac.uk/iupac/medchem/ (accessed on 8 March 2004)

### Prodrug

A prodrug is any compound that undergoes biotransformation before exhibiting its pharmacological effects. Prodrugs can thus be viewed as drugs containing specialized non-toxic protective groups used in a transient manner to alter or to eliminate undesirable properties in the parent molecule.

### Double prodrug (or pro-prodrug)

A double prodrug is a biologically inactive molecule which is transformed *in vivo* in two steps (enzymatically and/or chemically) to the active species.

### Carrier-linked prodrug (Carrier prodrug)

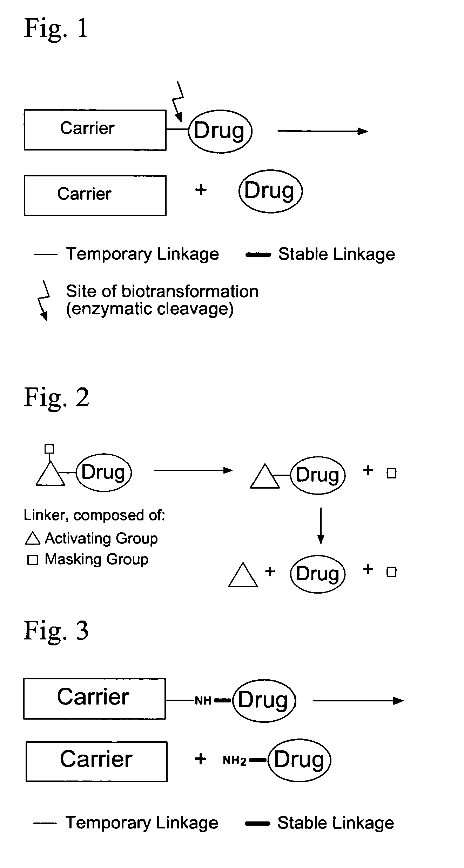

A carrier-linked prodrug is a prodrug that contains a temporary linkage of a given active substance with a transient carrier group that produces improved physicochemical or pharmacokinetic properties and that can be easily removed in vivo, usually by a hydrolytic cleavage. This is shown graphically in Fig. 1.

### Cascade prodrug

A cascade prodrug is a prodrug for which the cleavage of the carrier group becomes effective only after unmasking an activating group. This is shown graphically in Fig. 2.

### Bioprecursor prodrug

A bioprecursor prodrug is a prodrug that does not imply the linkage to a carrier group, but results from a molecular modification of the active principle itself. This modification generates a new compound, able to be transformed metabolically or chemically, the resulting compound being the active principle.

### Biotransformation

Biotransformation is the chemical conversion of substances by living organisms or enzyme preparations.

This invention applies in particular to carrier prodrugs and cascade prodrugs.
In polymeric prodrugs, the biologically active moieties are often linked to a polymeric carrier moiety by a labile bridge formed between the carrier moiety and a hydroxy, amino or carboxy group of the drug molecule.

Cleavage of a carrier prodrug generates a molecular entity (drug) of increased bioactivity and at least one side product, the carrier. This side product may be biologically inert (for instance PEG) or may have targeting properties (for instance antibodies). After cleavage, the bioactive entity will reveal at least one previously conjugated and thereby masked or protected functional group, and the presence of this group typically contributes to the bioactivity. Numerous macromolecular prodrugs are described in the literature where the macromolecular carrier is linked via a labile ester group to the medicinal agent (e.g. Y. Luo, MR Ziebell, GD Prestwich, "A Hyaluronic Acid - Taxol Antitumor Bioconjugate Targeted to Cancer Cells", Biomacromolecules 2000, 1, 208-218, J Cheng et al, Synthesis of Linear, beta-Cyclodextrin Based Polymers and Their Camptothecin Conjugates, Bioconjugate Chem. 2003, 14, 1007-1017, R. Bhatt et al, Synthesis and in Vivo Antitumor Activity of Poly(L-glutamic acid) Conjugates of 20(S)-Campththecin, J. Med. Chem. 2003, 46, 190-193 ; R.B. Greenwald, A. Pendri, C.D. Conover, H. Zhao, Y.H. Choe, A. Martinez, K. Shum, S. Guan, J. Med. Chem., 1999, 42, 3657-3667; B. Testa, J.M: Mayer in Hydrolysis in Drug and Prodrug Metabolism, Wiley-VCH, 2003,Chapter 8) In theses cases, the conjugated functional group of the bioactive entity is a hydroxyl group or a carboxylic acid.

Especially for biomacromolecules but also for small molecule polymer prodrugs, it may be desirable to link the macromolecular carrier to amino groups (i.e. N-terminus or lysine amino groups of proteins) of the bioactive entity, as is shown in Fig. 3. This will be the case if masking the drug's bioactivity requires conjugation of a certain amino group of the bioactive entity, for instance an amino group located in an active center or a region or epitope involved in receptor binding. Also, during preparation of the prodrug, amino groups may be more chemoselectively addressed and serve as a better handle for conjugating carrier and drug because of their greater nucleophilicity as compared to hydroxylic or phenolic groups. This is particularly true for proteins which may contain a great variety of different reactive functionalities. In this case non-selective conjugation reactions lead to undesired product mixtures which require extensive characterization or purification and may decrease reaction yield and therapeutic efficiency of the product.

Prodrug activation may occur by enzymatic or non-enzymatic cleavage of the labile bridge between the carrier and the drug molecule, or a sequential combination of both, i.e. an enzymatic step followed by a nonenzymatic rearrangement.

In an enzyme-free in vitro environment such as an aqueous buffer solution, almost any prodrug will undergo hydrolysis if labile linkages such as ester or amide bonds are present in the molecule. This rate of hydrolysis may be very slow and not therapeutically useful. In an in vivo environment, esterases or amidases are typically present and may cause significant catalytic acceleration of the kinetics of hydrolysis from twofold up to several orders of magnitude (see, for example, R.B. Greenwald et al. J.Med.Chem. 1999, 42 (18), 3857-3867).

The labile bond may be engineered for enzyme selectivity for organ or cell-type targeting. Targeted release of the bioactive entity is effected, if an enzyme, that selectively cleaves the linkage, is specifically present in the organ or cell type chosen for treatment. Several examples have been published for the prodrug activation of amine containing biologically active moiety by specific enzymes. In these cases, targeted release was the objective. G. Cavallaro et al., Bioconjugate Chem. 2001, 12, 143-151 described the enzymatic release of an antitumoral agent by the protease plasmin. Cytarabin was coupled via the tripeptide sequence D-Val-Leu-Lys to the polymer alpha, beta-poly(N-hydroxyethyl)-DL-aspartamide (PHEA). Enzymatic release of cytarabin was effected by the protease plasmin which concentration is relatively high in various kinds of tumor mass.

Further examples for antitumoral polymeric prodrugs activated by specific enzymes like beta lactamase (R. Satchi-Fainaro et al., Bioconjugate Chem. 2003, 14, 797-804) and cysteine proteases like cathepsin B (R. Duncan et al. J. Contr. Release 2001, 74, 135-146) have been described. Wiwattanapatapee et al. (2003) described a dendrimer prodrug for colonic delivery of 5-aminosalicylic acid. The drug molecule was conjugated by an azo bond to generation 3 PAMAM dendrimer. 5-aminosalicylic acid is released in the colon by a bacterial enzyme called azo reductase (W. R. Wiwattanapatapee, L. Lomlim, K. Saramunee, J. Controlled Release, 2003, 88: 1-9).

A.J. Garman et al. (A.J. Garman, S.B. Kalindjan, FEBS Lett. 1987, 223 (2), 361-365 1987) used PEG5000-maleic anhydride for the reversible modification of amino groups in tissue-type plasminogen activator and urokinase. Regeneration of functional enzyme from PEG-uPA conjugate upon incubation at pH 7.4 buffer by cleavage of the maleamic acid linkeage followed first order kinetics with a half-life of 6.1 h. The prodrug cleavage was not investigated in the presence of enzymes, and it can be expected - as explained above - that in vivo proteases will significantly contribute to the cleavage of the labile amide linkage. A further disadvantage of this linkage is the lack of stability of the conjugate at lower pH values that limits this approach to active agents stable at basic pH values as purification of the active agent polymer conjugate has to be performed under basic conditions.

R.B. Greenwald, A. Pendri, C.D. Conover, H. Zhao, Y.H. Choe, A. Martinez, K. Shum, S. Guan, J. Med. Chem., 1999, 42, 3657-3667 & PCT Patent Application WO-A-99/30727 described a methodology for synthesizing poly(ethylene glycol) prodrugs of amino-containing small molecule compounds based on 1,4- or 1,6-benzyl elimination. In this approach the amino group of the drug molecule is linked via a carbamate group to a PEGylated benzyl moiety. The poly(ethylene glycol) was attached to the benzyl group by ester, carbonate, carbamate, or amide bonds. The release of PEG from the drug molecule relied on the enzymatic cleavage of these masking groups. The cleavage of the release-triggering masking group is followed in this approach by the classical and rapid 1,4- or 1,6-benzyl elimination. As a consequence the highly reactive and potentially toxic quinone methide or quinonimine methide is liberated.

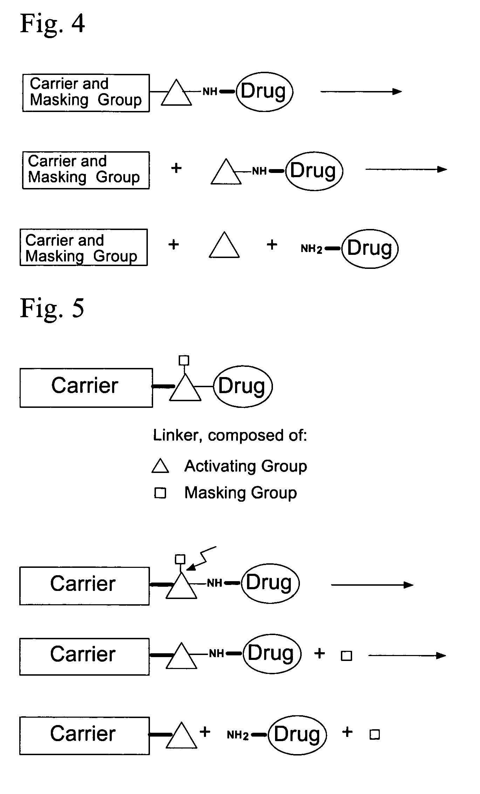

This is an example for a cascade carrier prodrug where the masking group is identical with the carrier and is shown diagrammatically in Fig. 4.

This linker system was also used for releasable poly(ethylene glycol) conjugates of proteins (S. Lee, R.B. Greenwald et al. Bioconj. Chem. 2001, 12 (2), 163-169). Lysozyme was used as model protein because it loses its activity when PEGylation takes place on the epsilon-amino group of lysine residues. Various amounts of PEG linker were conjugated to the protein. Regeneration of native protein from the PEG conjugates occurred by enzymatic cleavage in rat plasma or in non-physiological high pH buffer.

Greenwald et al. published in 2000 a poly(ethylene glycol) drug delivery system of amino-containing prodrugs based on trimethyl lock lactonization (R.B. Greenwald et al. J.Med.Chem. 2000, 43(3), 457-487; PCT Patent Application No. WO-A-02/089789). In this prodrug system substituted o-hydroxyphenyl-dimethylpropionic acid is coupled to amino groups of drug molecules by an amide bond. The hydroxy group is linked to PEG by an ester, carbonate, or carbamate group. The rate determining step in drug release is the enzymatic cleavage of these functional groups followed by fast amide cleavage by lactonization, liberating a potentially toxic aromatic lactone side product.

A common disadvantage of the linker systems described by Greenwald is the release of a potentially toxic aromatic small molecule side product after cleavage of these prodrug linker molecules.

More recently, R.B. Greenwald et al. (Greenwald et al. J. Med.Chem. 2004, 47, 726-734) described a PEG prodrug system based on bis-(N-2-hydroxyethyl)glycin amide (bicin amide) linker. In this system two PEG molecules are linked to a bicin molecule coupled to an amino group of the drug molecule. The first two steps in prodrug activation is the enzymatic cleavage of both PEG molecules. Different linkages between PEG and bicin are described resulting in different prodrug activation kinetics. The main disadvantage of this system is the slow hydrolysis rate of bicin amide conjugated to the drug molecule (tl/2 = 3 h in phosphate buffer) resulting in the release of a bicin-modified prodrug intermediate that may show different pharmacokinetic and pharmacodynamic properties compared to the parent drug molecule.

The in vivo release of stable linker-drug intermediates or potentially toxic side products can be avoided, if the carrier is not serving as a masking group and the activating group is coupled to the carrier by means of a stable bond. Antczak et al. (Bioorg Med Chem 9 (2001) 2843-48) describe a reagent which forms the basis for a cascade prodrug system for amine-containing drug molecules. In this approach an antibody serves as carrier, a stable bond connects the antibody to an activating moiety, carrying an enzymatically cleavable masking group. Upon enzymatic removal of the masking group, a second labile bond cleaves and releases the drug compound, as shown in Fig. 5.

A drawback of predominantly enzymatic cleavage is interpatient variability. Enzyme levels may differ significantly between individuals resulting in biological variation of prodrug activation by enzymatic cleavage. Enyzme levels may also vary depending on the site of administration, for instance it is known that in the case of subcutaneous injection, certain areas of the body yield more predictable therapeutic effects than others. To reduce this unpredictable effect, non-enzymatic cleavage or intramolecular catalysis is of particular interest (see, for example, B. Testa, J.M: Mayer in Hydrolysis in Drug and Prodrug Metabolism, Wiley-VCH, 2003, page 5).

Using enzyme specificity to control both targeting and release kinetics is a compromise. Enzyme-dependent release results in interpatient variability. Targeting through enzyme selectivity is of lower efficiency than for instance targeting through antibodies and may be achieved by more reliable means for instance by introducing certain structural features into the polymer moiety of the prodrug.

For these reasons, there is a need to provide novel linker and/or carrier technologies for forming polymeric prodrugs of amine containing active agents in order to overcome the limitations of the described polymeric prodrugs.

### Detailed description of the invention

The present invention addresses the disadvantages described above. The invention provides for a polymeric prodrug of Formula Ia or Ib. 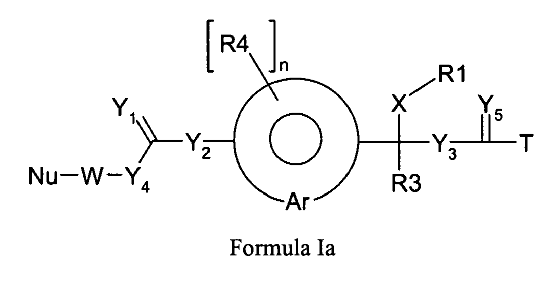 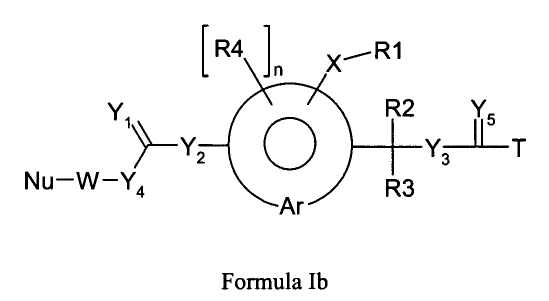 wherein Y₁ to Y₅, R1 to R4, T, X, W, Nu and Ar are defined below:

Native drug release is effected by a two step mechanism. The first step is the rate-determining cleavage of the masking group: 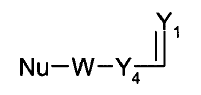 from the polymeric prodrug in vivo.

As described above, cleavage of the masking group may be mediated by an enzymatic or a non-enzymatic step, such as pH-dependent hydrolysis or intramolecular cyclization. In the preferred embodiment of the invention, cleavage is effected non-enzymatically by intramolecular cyclization or catalysis. The half-live of the cleaveage kinetics in an aqueous buffer of pH 7.4 at 37 °C of the masking group according to the present invention is preferably between 1 hour and 6 months, more preferably between 1 day and 3 months, and most preferably between 1 day and 2 months.

The second and final step in release of the regenerated native drug is the fast, spontaneous and irreversible so-called 1,4- or 1,6 or 1,(4+2p) (in which p = 2, 3, 4 or higher) elimination of the 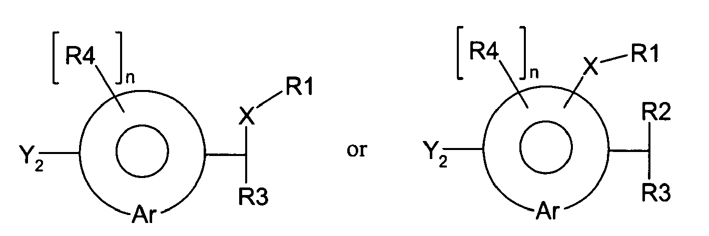 moiety of the remaining polymeric prodrug of formula Ia or formula Ib, respectively.

This mechanism of native drug release from a polymeric prodrug triggered by hydrolytic cleavage of the masking group followed by a 1,6-elimination step of the activating group is exemplified by an example of a polymeric prodrug according to the present invention.

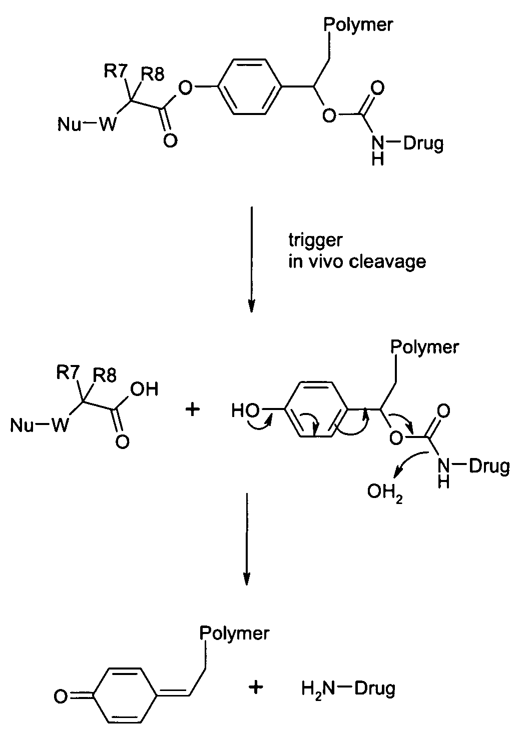

### Definition of Y₁ to Y₅, R1 to R4, T, X, W, Nu and Ar in formula Ia or Ib

T is D or A
and D is a residue of an amine-containing biologically active material including but not limited to small molecule bioactive agents or biopolymers like proteins, polypeptides and oligonucleotides (RNA, DNA), peptide nucleic acids (PNA), and A is a leaving group.

Non-limiting examples of suitable leaving groups A include but are not limited to chloride, bromide, fluoride, nitrophenoxy, imidazolyl, N-hydroxysuccinimidyl, N-hydroxybenzotriazolyl, N-hydroxyazobenzotriazolyl, pentafluorphenoxy, N-hydroxysulfosuccinimidyl, or any other leaving group known by those skilled in the art.

Suitable organic small molecule bioactive moieties include, without limitation, moieties such as central nervous system-active agents, anti-infective, anti-neoplastic, antibacterial, anti-fungal, analgesic, contraceptive, anti-inflammatory, steroidal, vasodilating, vasoconstricting, and cardiovascular agents with at least one primary or secondary amino group. Non-exclusive examples of such compounds are daunorubicin, doxorubicin, idarubicin, mitoxantron, aminoglutethimide, amantadine, diaphenylsulfon, ethambutol, sulfadiazin, sulfamerazin, sulfamethoxazol, sulfalen, clinafloxacin, moxifloxacin, ciprofloxaxin, enoxacin, norfloxacin, neomycin B, sprectinomycin, kanamycin A, meropenem, dopamin, dobutamin, lisinopril, serotonin, carbutamid, acivicin, etc.

Suitable proteins and polypeptides having at least one free amino group include but are not limited to ACTH, adenosine deaminase, agalsidase, albumin, alfa-1 antitrypsin (AAT), alfa-1 proteinase inhibitor (API), alteplase, anistreplase, ancrod serine protease, antibodies (monoclonal or polyclonal, and fragments or fusions), antithrombin III, antitrypsins, aprotinin, asparaginases, biphalin, bone-morphogenic proteins, calcitonin (salmon), collagenase, DNase, endorphins, enfuvirtide, enkephalins, erythropoietins, factor VIIa, factor VIII, factor VIIIa, factor IX, fibrinolysin, fusion proteins, follicle-stimulating hormones, granulocyte colony stimulating factor (G-CSF), galactosidase, glucagon, glucocerebrosidase, granulocyte macrophage colony stimulating factor (GM-CSF), phospholipase-activating protein (PLAP), gonadotropin chorionic (hCG), hemoglobins, hepatitis B vaccines, hirudin, hyaluronidases, idurnonidase, immune globulins, influenza vaccines, interleukins (1 alfa, 1 beta, 2, 3, 4, 6, 10, 11, 12), IL-1 receptor antagonist (rhIL-1ra), insulins, interferons (alfa 2a, alfa 2b, alfa 2c, beta 1a, beta 1b, gamma 1a, gamma 1b), keratinocyte growth factor (KGF), transforming growth factors, lactase, leuprolide, levothyroxine, luteinizing hormone, lyme vaccine, natriuretic peptide, pancrelipase, papain, parathyroid hormone, PDGF, pepsin, platelet activating factor acetylhydrolase (PAF-AH), prolactin, protein C, octreotide, secretin, sermorelin, superoxide dismutase (SOD), somatropins (growth hormone), somatostatin, streptokinase, sucrase, tetanus toxin fragment, tilactase, thrombins, thymosin, thyroid stimulating hormone, thyrotropin, tumor necrosis factor (TNF), TNF receptor-IgG Fc, tissue plasminogen activator (tPA), TSH, urate oxidase, urokinase, vaccines, plant proteins such as lectins and ricins.

Also included herein is any synthetic polypeptide or any portion of a polypeptide with in vivo bioactivity. Furthermore, proteins prepared by recombinant DNA methodologies including mutant versions of aforementioned proteins, antibody fragments, single chain binding proteins, catalytic antibodies and fusion proteins are included.

Preferred proteins are antibodies, calcitonin, G-CSF, GM-CSF, erythropoietins, hemoglobins, interleukins, insulins, interferons, SOD, somatropin, TNF, TNF-receptor-IgG Fc.

### X is a spacer moiety such as R5-Y6

Y₁, Y₂ can each be either O, S, or NR6, independently of each other.
Y₃, Y₅ can each be either O or S, independently of each other.
Y₄ is O, NR6, or -C(R7)(R8)-
Y₆ is O, S, NR6, succinimide, maleimide, unsaturated carbon-carbon bonds or any heteratom containing a free electron pair, or is not present.
R2 and R3 are selected independently from hydrogen, substituted or non-substituted linear, branched or cyclical alkyl or heteroalkyl, aryls, substituted aryls, substituted or non-substituted heteroaryls, cyano, nitro, halogen, carboxy, carboxyalkyl, alkylcarbonyl, carboxamidoalkyl, etc.

The term "heteroalkyl" in the context of the present invention denotes (linear, cyclical or branched) alkyl chains where the alkyl chains contain one or more heteroatoms, selected independently from O, S, N, P, Si, etc. or groups, selected independently from amide, ester, phosphonate ester, phosphate ester, double or triple bonds, carbamate, urea, thiourea, thiocarbamate, oxime, etc.

Each R4 substitution on Ar may be the same or different and is selected independently from hydrogen, substituted or non-substituted linear, branched or cyclical alkyl or heteroalkyl, aryl, substituted aryl, substituted or non-substituted heteroaryl, substituted or non-substituted linear, branched, or cyclical alkoxy, substituted or non-substituted linear, branched, or cyclical heteroalkyloxy, aryloxy, heteroaryloxy, etc.

R4 is selected preferably from small substituents such as hydrogen, methyl, ethyl, ethoxy, methoxy, and other C 1 to C6 linear, cyclical or branched alkyls and heteroalkyls.

n is zero or a positive integer.

R7 and R8 are selected independently from hydrogen, substituted or non-substituted linear, branched or cyclical alkyl or heteroalkyl, aryls, substituted aryls, substituted or non-substituted heteroaryls, carboxyalkyl, alkylcarbonyl, carboxamidoalkyl, cyano, halogen, etc.

R5 is selected from substituted or non-substituted linear, branched or cyclical alkyl or heteroalkyl, aryls, substituted aryls, substituted or non-substituted heteroaryls, carboxyalkyl, alkylcarbonyl, carboxamidoalkyl, etc.

R6 is selected from hydrogen, substituted or non-substituted linear, branched or cyclical alkyl or heteroalkyl, aryls, substituted aryls, substituted or non-substituted heteroaryls, etc.

R1 is a polymer.
Non-limiting examples for suitable polymers are polyalkyloxy-based polymers like poly(propylene glycol) or poly(ethylene glycol), dextran, chitosan, hyaluronic acid and derivatives, alginate, xylan, mannan, carrageenan, agarose, cellulose, starch, hydroxyethyl starch (HES) and other carbohydrate-based polmers, poly(vinyl alcohols), poly(oxazolines), poly(anhydrides), poly(ortho esters), poly(carbonates), poly(urethanes), poly(acrylic acids), poly(acrylamides) such as poly(hydroxypropylmethacrylamide) (HMPA), poly(acrylates), poly(methacrylates) like poly(hydroxyethylmethacrylate), poly(organophosphazenes), poly(siloxanes), poly(vinylpyrrolidone), poly(cyanoacrylates), poly(esters) such as poly(lactic acid) or poly(glycolic acids), poly(iminocarbonates), poly(amino acids) such as poly(glutamic acid), collagen, gelatin, copolymers, grafted copolymers, cross-linked polymers, hydrogels, and block copolymers from the above listed polymers.

Hydrogels may be defined as three-dimensional, hydrophilic or amphiphilic polymeric networks imbibing large quantities of water. The networks are composed of homopolymers or copolymers, are insoluble due to the presence of covalent chemical or physical (ionic, hydrophobic interactions, entanglements) crosslinks. The crosslinks provide the network structure and physical integrity. Hydrogels exhibit a thermodynamic compatibility with water which allows them to swell in aqueous media.(see.: N.A. Peppas, P. Bures, W. Leobandung, H. Ichikawa, Hydrogels in pharmaceutical formulations, Eur. J. Pharm. Biopharm. 2000, 50, 27-46). The chains of the network are connected in such a fashion that pores exist and that a substantial fraction of these pores are of dimensions of between 1 and 1000 nm. By selecting certain polymerization conditions, the hydrogel may be obtained in the form of an amorphous gel or as beaded resin. Such soft beads may have a diameter of between 1 and 1000 micrometer.

Hydrogels may be synthesized from the polymers and copolymers listed above and physically cross-linked or chemically cross-linked by radical, anionic or cationic polymerization, by chemical reactions like condensation or addition reactions as described in W.E. Hennink and C.F. van Nostrum, Adv. Drug Del. Rev. 2002, 54, 13-36.

Further examples include branched and hyperbranched polymers. Examples for such polymers include dendrimers and other dense star polymers. (R. Esfand, D.A. Tomalia, Drug Discov Today, 2001, 6(8), 427-436; P.M. Heegaard, U. Boas, Chem. Soc. Rev. 2004 (33(1), 43-63; S.M. Grayson, J.M. Frechet, Chem. Rev. 2001, 101 (12), 3819-3868).

R1 can also be a biopolymer like a protein. Non-limiting examples of such polymers include albumin, antibodies, fibrin, casein, and other plasma proteins.

Each R1 polymer can carry one or more biologically active substances linked to the polymer by the prodrug linker described herein or any other linker known to the person skilled in the art. The polymers may have further substituents and may be functionalized for attachment to the spacer moiety X. Non-limiting examples of such functional groups comprise carboxylic acid and activated derivatives, amino, maleimide, thiol, sulfonic acid and derivatives, carbonate and derivatives, carbamate and derivatives, hydroxyl , aldehyde, ketone, hydrazine , isocyanate, isothiocyanate, phosphoric acid and derivatives, phosphonic acid and derivatives, haloacetyl, alkyl halides, acryloyl, arylating agents like aryl fluorides, hydroxylamine, disulfides like pyridyl disulfide, vinyl sulfone, vinyl ketone, diazoalkanes, diazoacetyl compounds, epoxide, oxirane, and aziridine.

Preferred functional groups for the R1 polymer include but are not limited to thiol, maleimide, amino, carboxylic acid and derivatives, carbonate and derivatives, carbamate and derivatives, aldehyde, and haloacetyl.

Especially preferred functional groups include thiol, maleimide, amino, carboxylic acid and derivatives, carbamate and derivatives, and carbonate and derivatives thereof.

Non-limiting examples for suitable bonds or groups formed between X and R1 include disulfide, S-succinimido, amide, amino, carboxylic ester, sulfonamide, carbamate, carbonate, ether, oxime, hydrazone, urea, thiourea , phosphate, phosphonate, etc.

Preferred bonds or groups formed between X and R1 comprise S-succinimido, amide, carbamate, and urea.

Preferably, the R1 polymers are well hydrated, degradable or excretable, nontoxic and non-immunogenic in mammals. Preferred R1 polymers include polyalkoxy-based polymers like polyethylene glycol and polyethylene glycol reagents as those described in Nektar Inc. 2003 catalog "Nektar Molecule Engineering - Polyethylene Glycol and Derivatives for Advanced PEGylation" and branched, hyperbranched, cross-linked polymers and hydrogels.

W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl, etc.

W is selected preferably from non-toxic substituted or non-substituted linear, branched or cyclical alkyls or heteroalkyls.

Preferred variations of 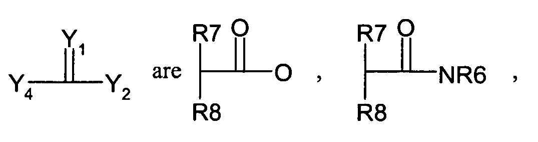 and

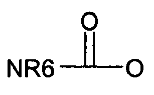

At least one Nu is present in Nu-W.

Nu is a nucleophile that can perform a nucleophilic attack at the carbonyl carbon of 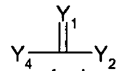 and thus catalyse the cleavage of the masking group by intramolecular catalysis or cyclization. Nucleophiles are any functional groups or heteroatoms carrying at least one free electron pair. Preferred nucleophiles include primary, secondary and tertiary amino groups, thiol, carboxylic acid, hydroxylamine, hydrazine. In order to effectively catalyse the cleavage of the masking group, the spacing between the nucleophile Nu and Y₂ is preferably between three and fifteen atoms. More preferably, the spacing betetween Nu and Y₂ is between four and eight atoms. The at least one nucleophile Nu may be attached anywhere to W (e.g. at the terminus or in the middle of W) or may be part of W.

An especially preferred variation of 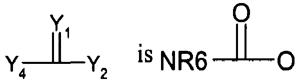 and R6 may also be Nu-W and is not hydrogen. In this case, preferred variations of each Nu-W are selected independently from substructures according to the following substructure: 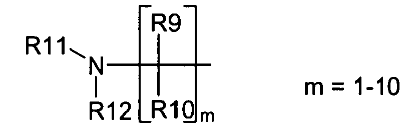 R9, R10, R11, and R12 are selected independently from hydrogen, substituted or non-substituted alkyl or heteroalkyl, substituted or non-substituted aryl or heteroaryl.

Preferably, R9, R10, R11, and R12 are selected independently from hydrogen, substituted or non-substituted alkyl.

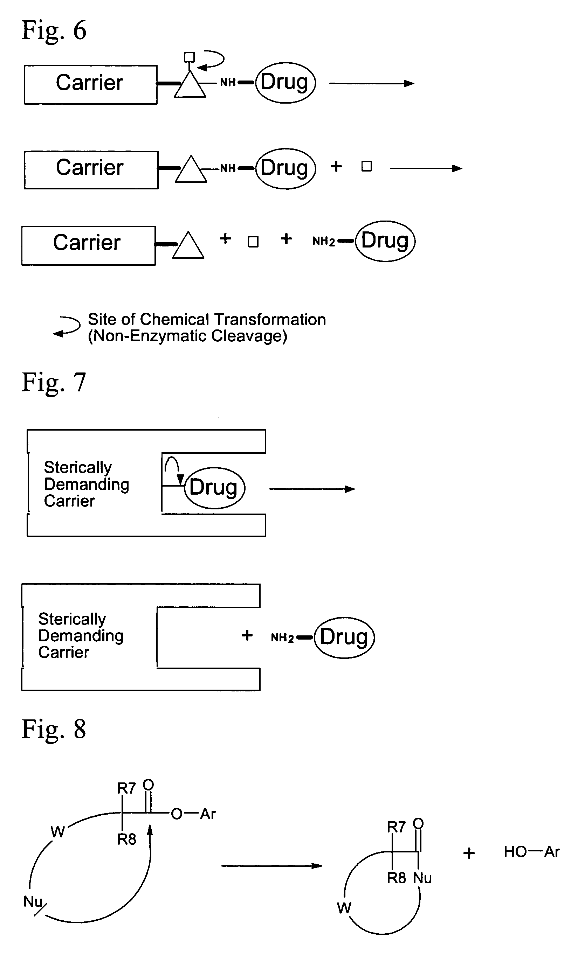

Figure 8 shows an example according to formula Ia or Ib wherein the cleavage of the masking group is by intramolecular cyclization.
In cases where Nu only catalyses the cleavage of the masking group by intramolecular catalysis, no cyclical product of the masking group is formed.

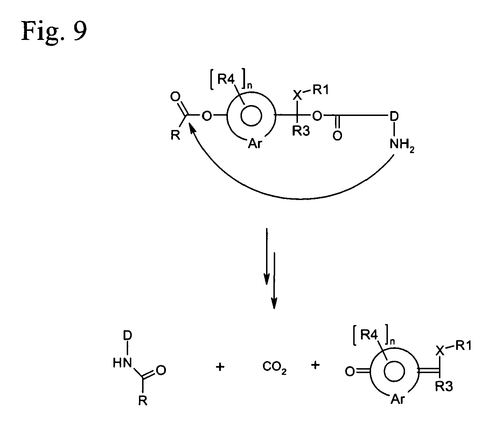

Surprisingly it was found, that the masking group can modify irreversibly the amine containing biologically active moiety when the nucleophile Nu is absent in the masking group. As shown in the example section, during release of the bioactive moiety insulin from a polymeric prodrug with a pentanoyl masking group which is not part of the present invention (as it does not contain a nucleotide), approximately 30 % of the insulin molecule was modified with the masking group by acyl transfer. The mechanism of an example of this modification where D contains an additional free amino group that serve as nucleophile for acyl transfer is shown in figure 9.

Ar of formula Ia or Ib is a multi-substituted aromatic hydrocarbon or a multi-substituted aromatic heterocycle. To be aromatic, the number of pi electrons must satisfy the Hückel rule (4n+2) and the cycle has to be planar. A huge variety of compounds satisfy these criteria and thus are suitable as Ar in formula Ia or Ib. Non-limiting preferred aromatic moieties include: 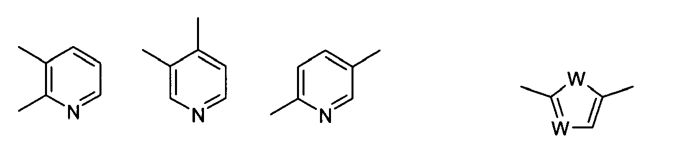 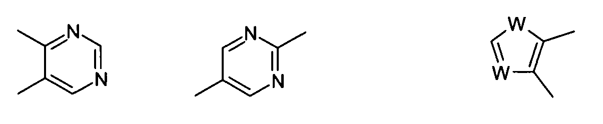 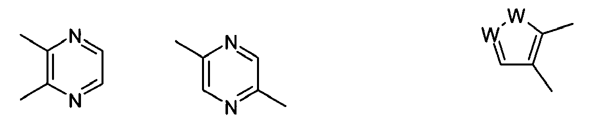 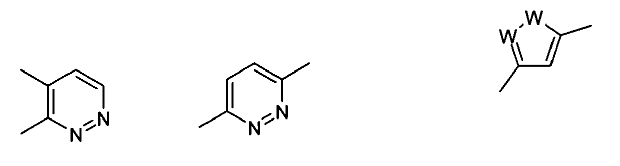 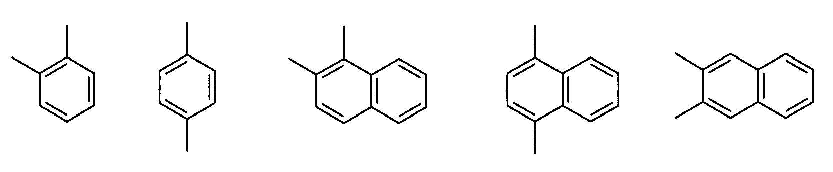 wherein W is O, N, or S, independent from each other.

Y₂ and 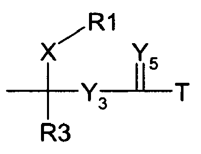 in formula Ia or Y₂ and 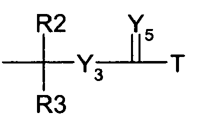 in formula Ib have to be arranged on the aromatic ring in such a fashion that a 1,4- or 1,6- or 1,(4+2p), with p= 2, 3, 4 and higher, elimination can take place (see above). For example, in the case of a 6-membered ring, the substituents have to be arranged *ortho* or *para.*

Preferred moieties for Ar are mono- and dicyclic aromatic hydrocarbons or aromatic heterocycles.

Especially preferred moieties are monocyclic five- or six-membered aromatic hydrocarbons or aromatic heterocycles.

### General synthesis procedures of the polymeric prodrugs

Synthesis of representative examples of polymeric prodrugs according to the present invention is described in the Examples section.

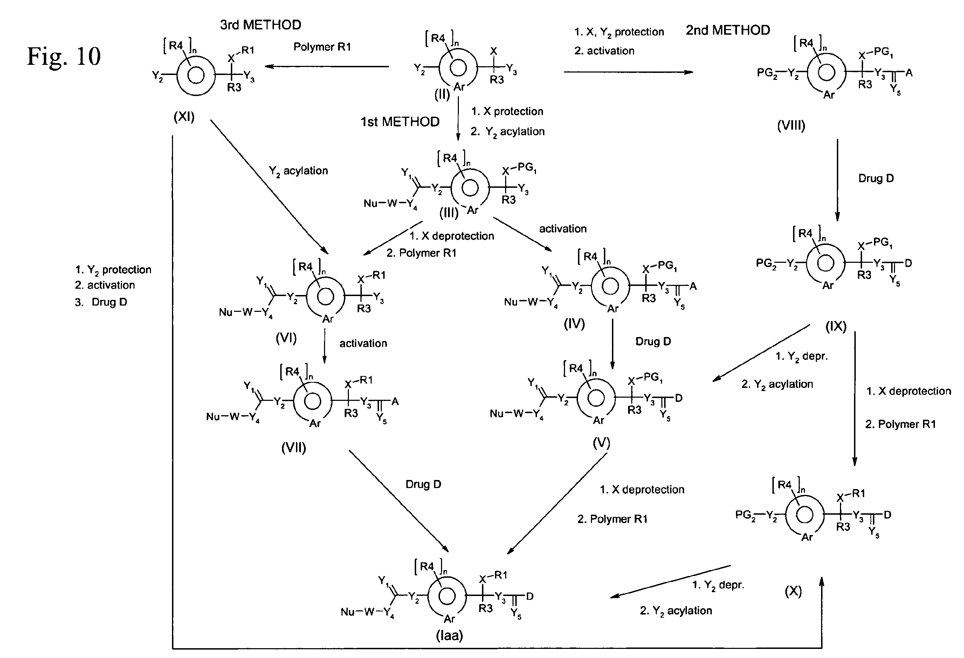

Prodrugs of the present invention can be prepared in various different fashions. Figure 10 shows general routes for the synthesis of the polymeric prodrugs of the present invention according to formula Ia.
In a first method, intermediate (III) is provided by acylating Y₂ of starting material (II) with

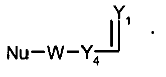

For this, X or Nu may have to be protected with a reversible protecting group PG₁. Suitable protecting groups are described in TW Greene, P.G.M. Wuts, Protective groups in organic synthesis, 1999, John Wiley & Sons, 3^{rd} ed.. From intermediate (III) two alternative routes can be used to yield (Iaa). In a first route intermediate (III) is activated by an activating agent such as 4-nitrophenyl chloroformate or disuccinyl carbonate to yield (IV). The amine containing drug molecule is attached to (IV) to yield (V) by displacing the leaving group of the activated intermediate (IV). After deprotection of X such as by treating intermediate (V) with reagents like trifluoroacetic acid or DTT (where applicable) deprotected intermediate (V) is then reacted with polymer R1 to yield the polymeric prodrug (Iaa).

In a second route the polymer R1 is first attached to the intermediate (III) after deprotection of X (where applicable) to form intermediate (VI). After an activation step intermediate (VII) is formed. (VII) is reacted with the amine containing drug molecule to form the polymeric prodrug (Iaa).

In a second method, intermediate (VIII) is provided by activating starting material (II) by an activating agent such as 4-nitrophenyl chlorofomate. For this, Y₂ and/or X may have to be protected with a protecting group PG₂ and/or PG₁. Amine containing drug is reacted with intermediate (VIII) to form (IX). In a first route, Y₂ of (IX) is selectively deprotected and acylated to form intermediate (V) which is further processed to (Iaa) as described above. In a second route X is selectively deprotected and reacted with polymer R1 to form intermediate (X). Y₂ of (X) is then deprotected and acylated to form the polymeric prodrug (Iaa).

In a third method starting material (II) is reacted with polymer R1 to form intermediate (XI). In one route, intermediate (XI) can be acylated to form intermediate (VI) which processed as described above to form polymeric prodrug (Iaa). In a second route, Y₂ is protected by the protecting group PG₂, activated and reacted with the amine containing drug molecule to form (X). Intermediate (X) is then processed as described above to form the polymeric prodrug (Iaa).

For all methods described, further functional groups such as Y₃ or nucleophiles present in Nu-W may have to be protected with suitable protecting groups.

Polymeric prodrugs according to formula Ib can be prepared by methods described above for prodrugs according to formula Ia using starting material IIb instead of II in figure 10.

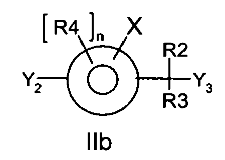

It is understood, that linker structures according to the outlined invention and carrying protecting groups or leaving groups as described and used in the synthesis of corresponding polymeric prodrugs are considered within the range of the invention.

### Application of the polymeric prodrugs in molecular therapy

One advantage of the described prodrug systems is to enable depot formulations without the need for encapsulation. Until now, many biocompatible materials like hydrogels with large pore sizes could not be used for depot formulations due to their lack of encapsulation properties. From such well-hydrated and mechanically soft biocompatible materials,biologically active moiety would be released too fast for most therapeutic applications. In combination with the prodrug linkers described in this invention, the carrier material may be optimized for its biocompatibility properties as the release is solely governed by the linker cleavage kinetics and does not require chemical or enzymatic degradation of the polymer carrier itself.

A second advantage of the polymeric prodrugs of the present invention is their predominantly non-enzymatic cleavage: the half-life of the prodrug in suitably buffered human blood plasma of pH 7.4 (with aqueous buffer concentration < 50 %) is at least 50 % of the half-life of the prodrug in enzyme-free buffer pH 7.4.

This feature allows for better predictability and control of release rates after administration to a living organism and reduces interpatient variability.

Release rates are governed by a substantially non-enzymatic chemical reaction which is in turn dependent on the molecular structure of the linker. Systematic or random modifications of the chemical structure, for instance by changing substituents in one or more positions, for instance a masking group in a cascade prodrug, allows for the generation of prodrung linkers with differing release rates. It is therefore possible to create a variety of prodrug linkers and select those fast or slow cleaving prodrug linkers according to the demands posed by a given medicinal or therapeutic application.

One embodiment of the invention relates to such cascade-prodrugs where the removal of a masking group from an activating moiety triggers a release sequence.

Another advantageous feature which is part of this invention is the attachment of the polymer carrier through a stable covalent bond to an activating moiety involved in a double or cascade prodrug release mechanism. Typically in polymer pro-prodrugs, a bond between the polymer carrier and the activating moiety is cleaved, this being the release-controlling step, followed by a more rapid molecular rearrangement of the activating moiety and cleavage of a second labile linkage which is located between the activating moiety and the drug. After completion of this sequence, the polymer carrier as well as the activating moiety are released in addition to the drug. Due to the rearrangement, the activating moiety is released in a highly reactive form and may cause direct damage to surrounding biomolecules, or potentially toxic derivatives of the activating moiety may be formed in vivo. As part of this invention, the activating moiety remains attached to the polymer carrier after drug release and therefore cannot diffuse into the environment. Polymer carrier attachment greatly reduces the reactivity of the activiating moiety and the probability of unwanted side effects.

In contrast to the enzymatic dependency of masking group removal as described by Antzczak, C., Bauvois, B., Monneret, C., Florent J-C, Bioorg Med Chem 9, 2001, 2843), a higher level of control over release rates can be achieved if the masking group has enzyme-independent, self-eliminating properties. The masking groups according to the present invention contain at least one nucleophile Nu. This nucleophile, which can be for example a primary, secondary or tertiary amino group can assist the cleavage of the masking group from the activating moiety by intramolecular catalysis or cyclization. For instance the masking group may contain an amine group attached to a four carbon atom chain, which in turn is linked through an ester group to the activating moiety. The free amine may react with this ester in an intramolecular fashion, resulting in ester cleavage, amidation and cyclization of the masking group, forming a six-membered ring. Structural features of this masking group such as nucleophilicity of the amine group and ring-forming capacity may be systematically optimized in order to precisely adjust the rate of prodrug cleavage. Such intramolecular reactions resulting in unmasking and subsequent rearrangement are highly independent from enzymes due to the fact that intramolecular reactions are generally preferred over intermolecular reactions and are shown diagrammatically in Fig.6.

In another embodiment of the invention, independency of prodrug cleavage from enzyme levels is achieved by providing a prodrug containing a sterically demanding carrier group as is shown in Fig. 7.

Such encapsulation or sterical protection by the sterically demanding carrier group may be conferred by a branched, hyperbranched, crosslinked or self-assembled structure of the carrier polymer. Such polymers tend to form a densely packed molecular volume, as exemplified for instance in dendrimers, dense star polymers or bead-shaped nano- and microparticles or amorphous gels. If the linkage of the polymer carrier to the drug is located in the interior of the polymer carrier, the linked drug will be efficiently encapsulated and protected from enzymatic attack. In this case, sterical hindrance by the polymer prevents enzymes from accessing and cleaving the labile linkage.

In yet another embodiment, enzyme-independent prodrug cleavage is achieved by combining an intramolecular self-eliminating masking group with an encapsulating hyperbranched or crosslinked or self-assembled carrier.

A further advantage of the present invention is the release of an unmodified biologically active moiety. In cases where the biologically active moiety contains further reactive functional groups like amino groups of lysine residues in proteins, an unwanted side reaction between the masking group and the biologically active moiety can occur. The reactive functional groups of the biologically active moiety may react with the masking group, forming a stable covalent bond and resulting in the release of a modified biologically active moiety. This potential side reaction is shown schematically in figure 9. The occurrence of such side reactions is shown in the examples section using polymeric prodrugs which are not part of the present invention with simple masking groups like a pentanoyl residue without a nucleophile Nu present in the masking group. This side reaction is suppressed using polymeric prodrugs according to the present invention with intramolecularly activated masking groups that contain nucleophiles Nu (see experimental section).

### Description of the Figures

Fig. 1 shows a carrier-linked prodrug.
Fig. 2 shows a cascade prodrug.
Fig. 3 shows cleavage of a prodrug with an amino group.
Fig. 4 shows produg cleavage from a carrier which is identical with a masking group.
Fig. 5 shows cleavage of a cascade prodrug system
Fig. 6 shows cleavage and intramolecular reaction of the masking group.
Fig. 7 shows cleavage of a prodrug having a sterically demanding carrier group.
Fig. 8 shows cleavage of the masker group by intramolecular cyclisation.
Fig. 9 shows a possible side reaction of polymeric prodrug activation
Fig.10 shows general synthesis methods
Fig.11 shows mass spectra of prodrug released insulin molecules

### Examples

### Materials

Fmoc-amino acids, resins and PyBOP were purchased from Novabiochem and are named according to the catalogue. Fmoc-Ado-OH was obtained from Neosystem. All additional chemicals were purchased from Sigma Aldrich. Recombinant human insulin was from ICN Biomedicals (USA). Maleimide-PEG5k was obtained from Nektar (USA). 5-(and-6)-carboxyfluorescein succinimidyl ester (mixed isomers) was obtained from Molecular Probes.

### Solid phase synthesis reaction medium

Solid phase synthesis was performed on NovaSyn TG Sieber amide resin with a loading of 0.17 mmol/g or 2-chlorotrityl chloride resin with a loading of 1.4 mmol/g. Syringes equipped with polypropylene frits were used as reaction vessels.

### Standard coupling cycle for fmoc-protected amino acids

For fmoc protecting-group removal, the resin was repeatedly (three times, 4 min each) agitated with 2/2/96 (v/v/v) piperidine/DBU/DMF and repeatedly (six times) washed with DMF.
Coupling of fmoc-protected amino acids to free amino groups on resin was achieved by agitating the resin with 3 equivalents (eq) of fmoc-amino acid, 3 eq PyBOP and 6 eq DIEA in relation to free amino groups in DMF for 60 min.
Finally, the resin was repeatedly (five times) washed with DMF.

### Standard cleavage protocol for TentaGel Sieber amide resin

Upon completed synthesis, the resin was washed with DCM, dried in vacuo and treated repeatedly (five times) with 97/2/1 (v/v) DCM/TES/TFA. After evaporation, compounds were purified by preparative RP-HPLC (Waters 600).

### Standard cleavage protocol for 2-chlorotrityl chloride resin

Upon completed synthesis, the resin was washed with DCM, dried in vacuo and treated two times for 30 minutes with 65/35 (v/v) HFIP/DCM. After combining the eluates the volatile components were evaporated.

### Analysis

Mass spectrometry (MS) was performed on a Waters ZQ 4000 ESI instrument and spectra were, if necessary, interpreted by Waters software MaxEnt.
Size exclusion chromatography was performed using an Amersham Bioscience AEKTAbasic system equipped with a Superdex 200 column (Amersham Bioscience).
NMR spectra were recorded on a Bruker AC300.

### Overview - synthesis of polymeric prodrugs according to formula Ia with ester-linked masking groups

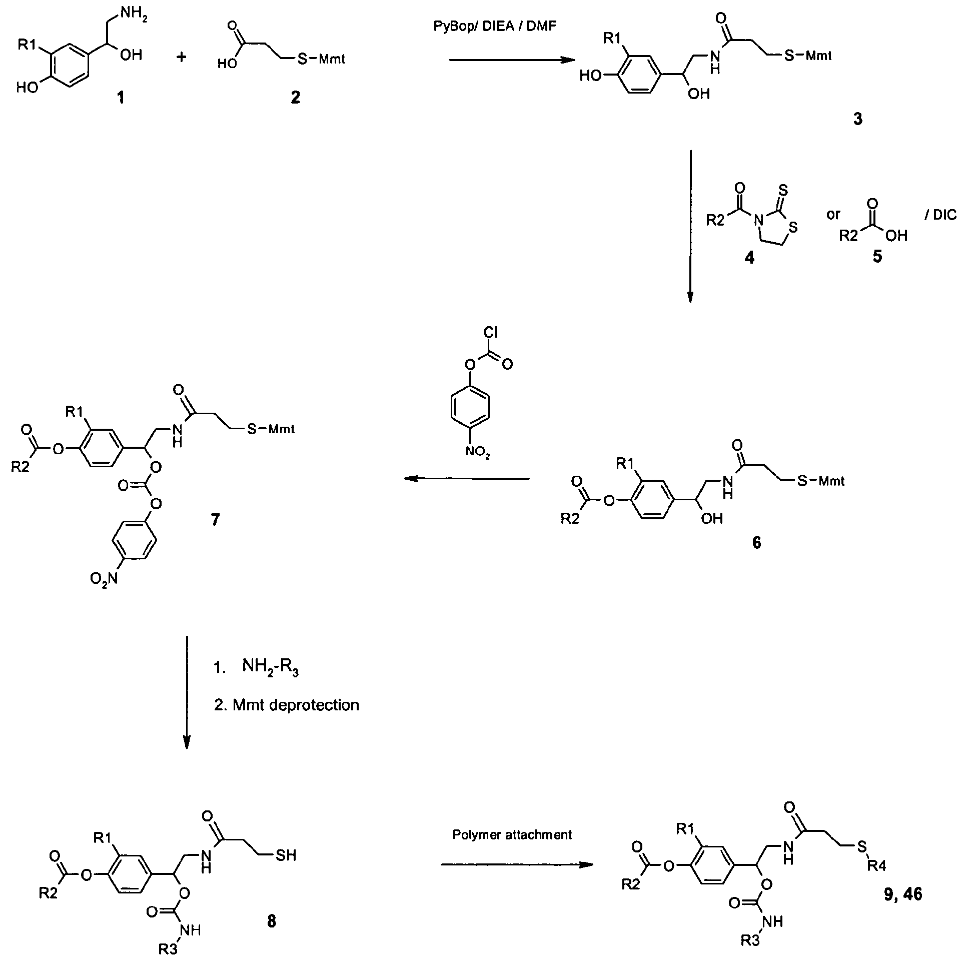

### Synthesis of polymeric prodrugs according to formula Ia with carbamate-linked masking groups

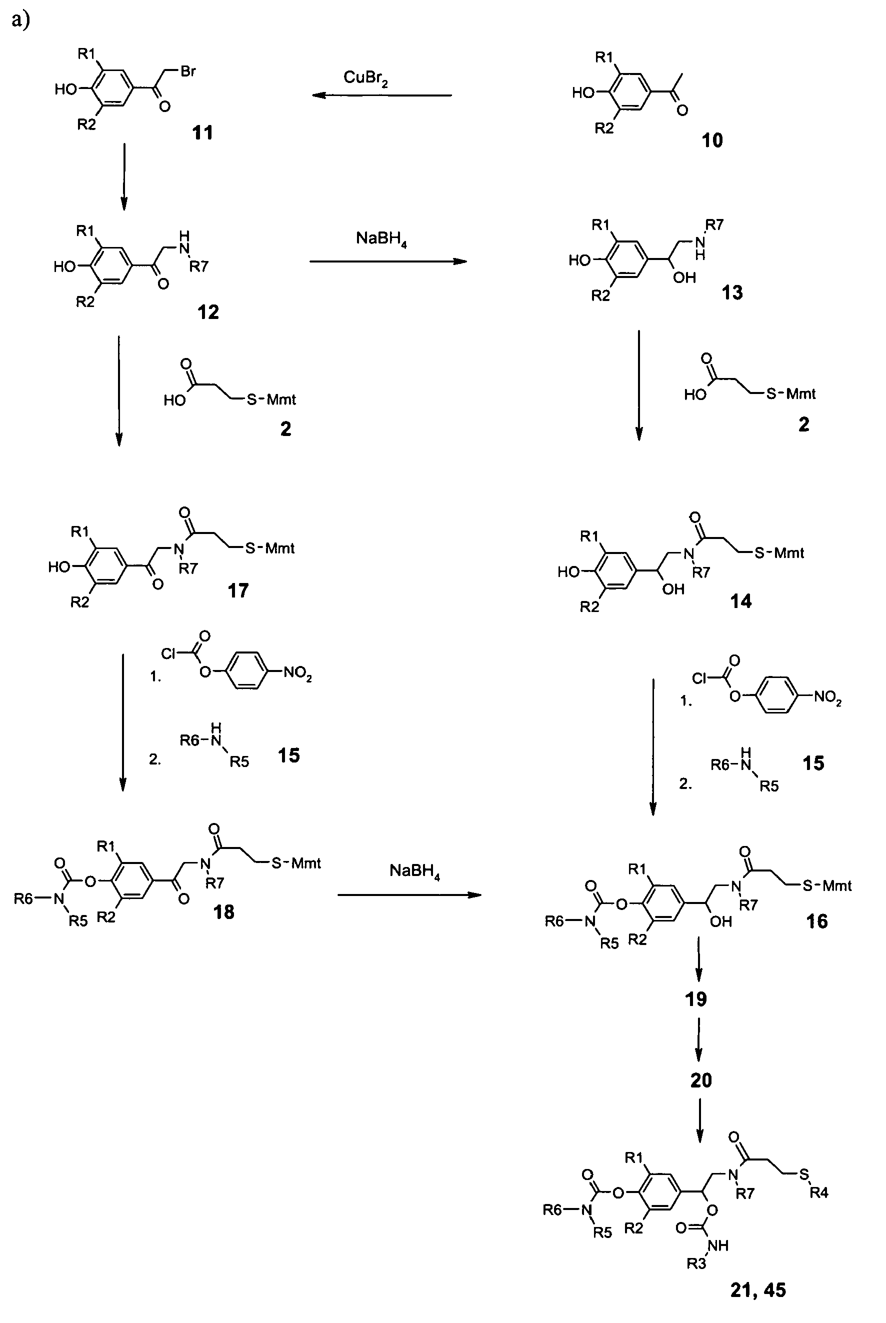

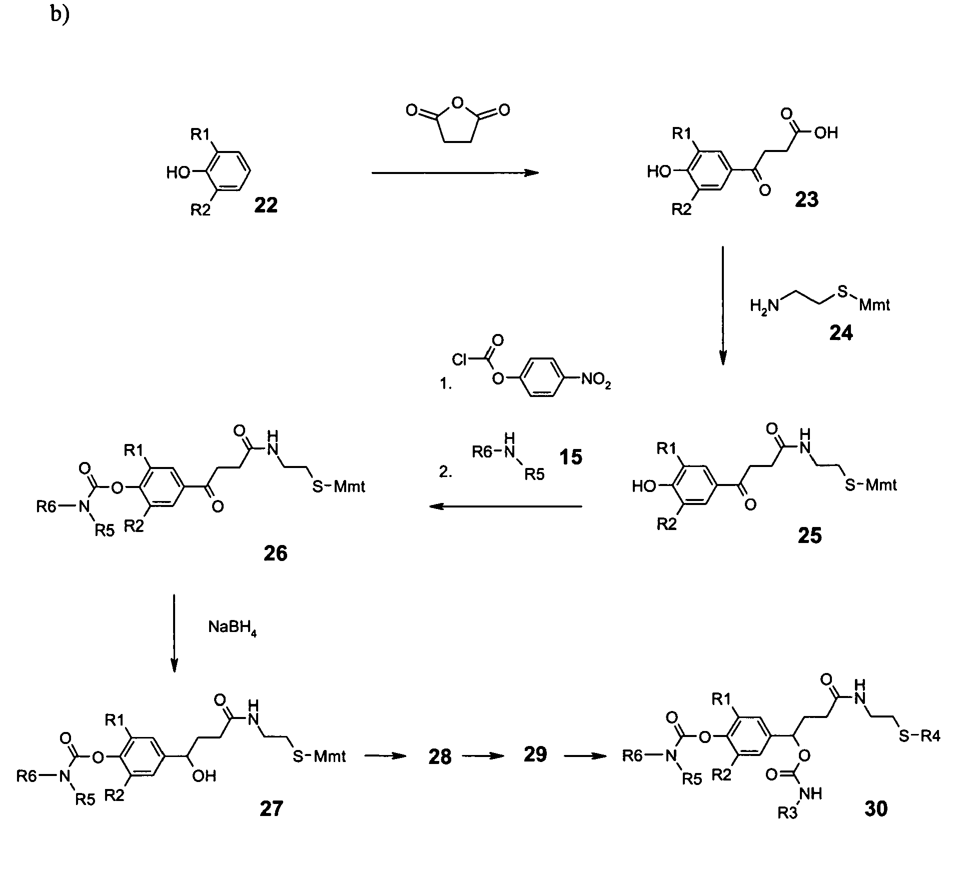

### Synthesis of polymeric prodrugs according to formula Ib with a carbamate-linked masking group

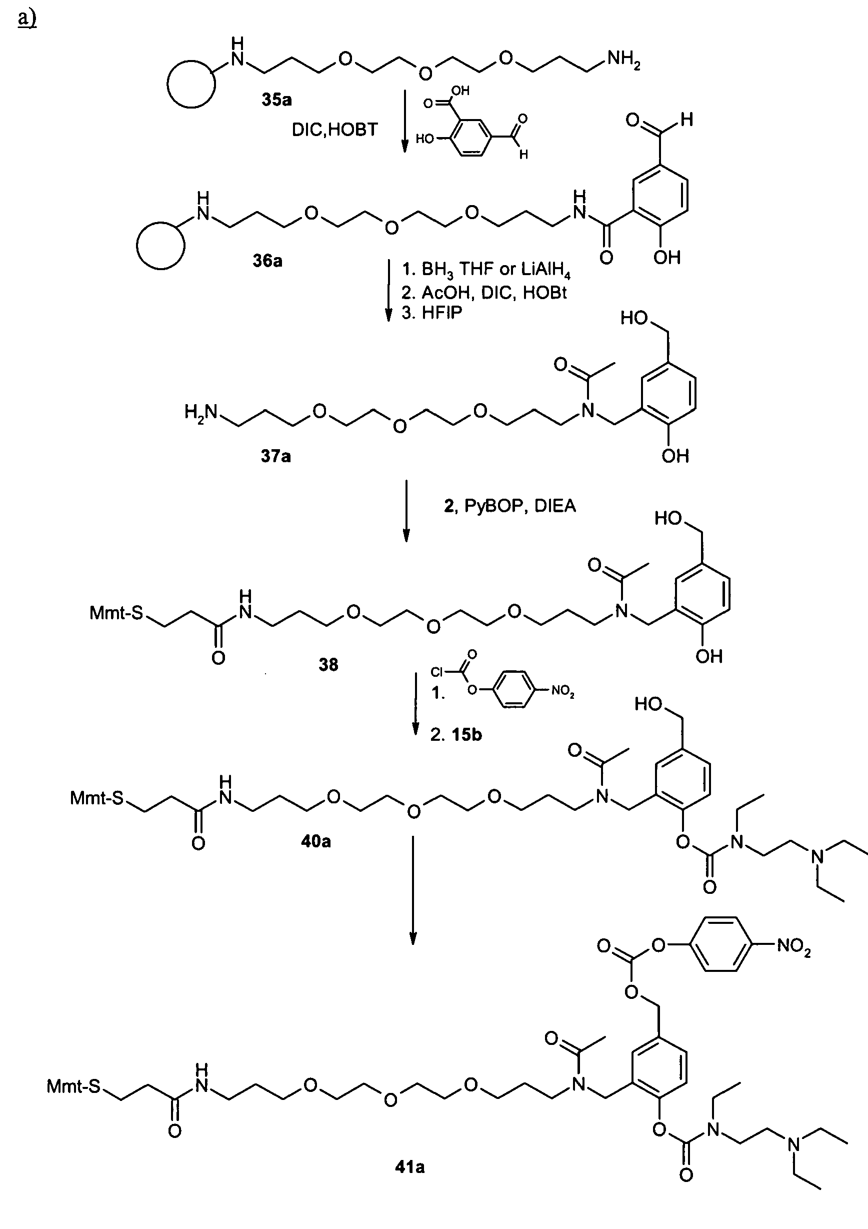

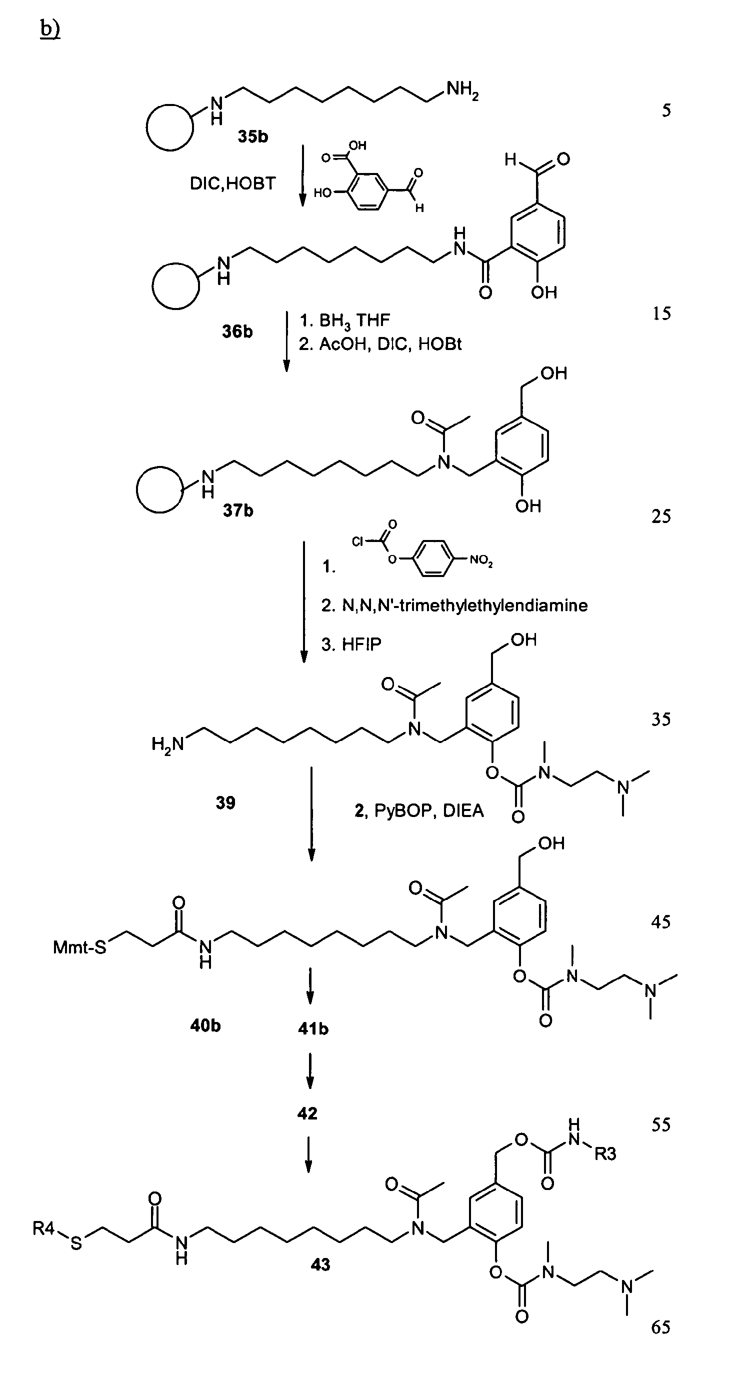

### Synthesis of compound 2

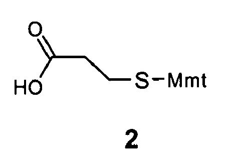

Mmt-chloride (1 eq) and mercaptopropionic acid (1.1 eq) were dissolved in TFA and incubated for 30 min. The solvent was removed under reduced pressure. The product was dissolved in pyridine, diluted in water, acidified by acetic acid and extracted with ether. The ether phase was separated and dried over Na₂SO₄. Solvent was removed under reduced pressure and product **2** was purified by RP-HPLC.

### Synthesis of compounds 3a and 3b

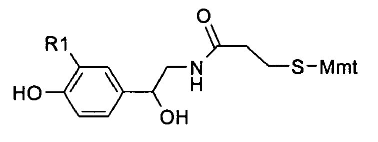

**3a** R1 = H
**3b** R1 = OMe

Octopamine hydrochloride **1a** or normetanephrine hydrochloride **1b** (2 eq), DIEA (4 eq), and PyBOP (1 eq) were dissolved in DMF, **2** (1eq) was added and the mixture was reacted for 50 min at room temperature. After addition of acetic acid (7 eq) products **3a** or **3b** were purified by RP-HPLC.

**3a:** MS [M+Na]⁺ = 536 (MW+Na calculated = 536.2 g/mol)
**3b:** MS [M+Na]⁺ = 566 (MW+Na calculated = 566.2 g/mol)

### Synthesis of mercaptothiazolide 4

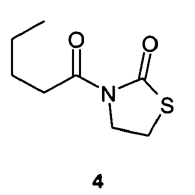

2-Mercaptothiazoline and triethylamine (1.5 eq) were dissolved in dry THF and pentanoyl chloride (1 eq) was added. The mixture was stirred for 1 h at 50 °C under inert gas atmosphere and was allowed to cool to room temperature. 0.5 N aqueous HCl was added and the separated organic phases were dried over Na₂SO₄. After concentration *in vacuo* the residue was purified by silica gel column chromatography using heptane/ethyl acetate (1/1) as mobile phase. Mercaptothiazolide **4** was collected as a viscous yellow oil.

**4** R_{f} (heptane/ethyl acetate 1:1) = 0.7

### Synthesis of compounds 5a and 5b

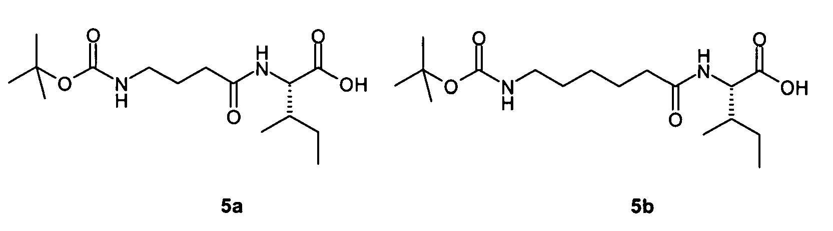

### General synthesis protocol:

1 g 2-chlorotrityl chloride resin (loading 1.6 mmol/g) was incubated for 1 h with 850 mg (2.4 mmol) Fmoc-Ile-OH and 840 µl (4.8 mmol) DIEA in 5 ml 1/1 DCM/DMF. After fmoc removal and washing of the resin with DMF, boc-aminobutyric acid or boc-aminohexanoic acid, respectively, were coupled to 0.5 g resin each according to the standard coupling method. Compounds **5a** or **5b,** respectively, were cleaved from the resin with 97/1/2 (v/v) DCM/TFA/TES for 45 min. After neutralisation with pyridine, solvents were removed under reduced pressure and **5a** or **5b** were purified by RP-HPLC.

**5a** MS [M+Na]⁺ = 339.2 (MW+Na calculated = 339.4 g/mol)
**5b** MS [M+Na]⁺ = 367.4 (MW+Na calculated = 367.5 g/mol)

### Synthesis of compound 6a

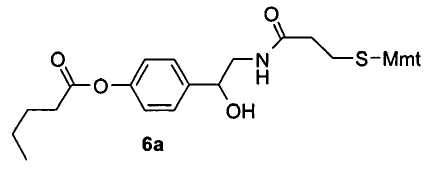

Mercaptothiazolide **4** (1 eq), phenol **3a** (4 eq) and DMAP (4 eq) were refluxed in DCM for 2 h under nitrogen atmosphere. After neutralization with acetic acid, the solvent was removed *in vacuo* and product **6a** was purified by RP-HPLC.

**6a** MS [M+Na]⁺ = 620 (MW+Na calculated = 620.3 g/mol)

### Synthesis of compounds 6b to 6e

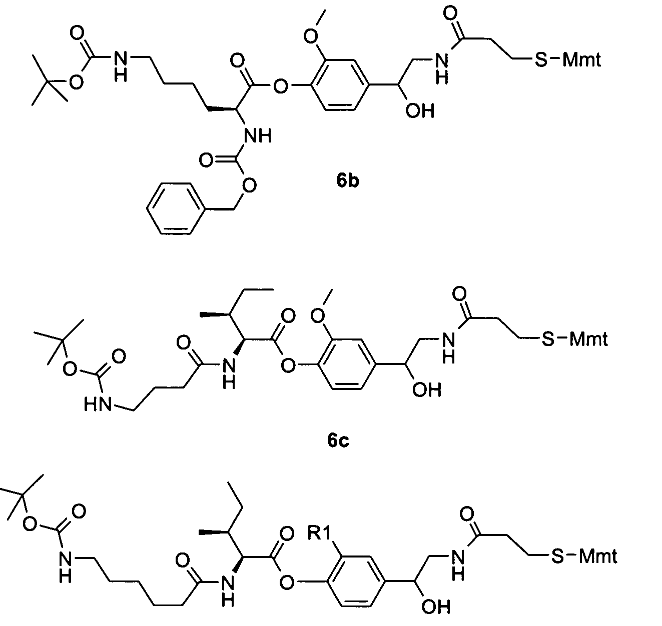

### General synthesis protocol:

**6d R1 = OMe**
**6e R1 = H**
Carboxylic acid **5a, 5b** or Z-Lys(Boc)-OH (1 eq), **phenol 3b** (1 eq), DIC (1 eq), and DMAP (2 eq) in DMF were reacted for 1h at room temperature. After addition of acetic acid (4 eq) the resulting carboxylic esters **6c, 6d** or **6b,** respectively, were purified by RP-HPLC.
**6e** was synthesized as described above using **5b** and **3a** as starting materials.

**6b** MS [M+Na]⁺ = 928 (MW+Na calculated = 928.6 g/mol)
**6c** MS [M+Na]⁺ = 864 (MW+Na calculated = 864.5 g/mol)
**6d** MS [M+Na]⁺ = 892 (MW+Na calculated = 892.6 g/mol)
**6e** MS [M+Na]⁺ = 862 (MW+Na calculated = 862.6 g/mol)

### Synthesis of compounds 7a to 7e

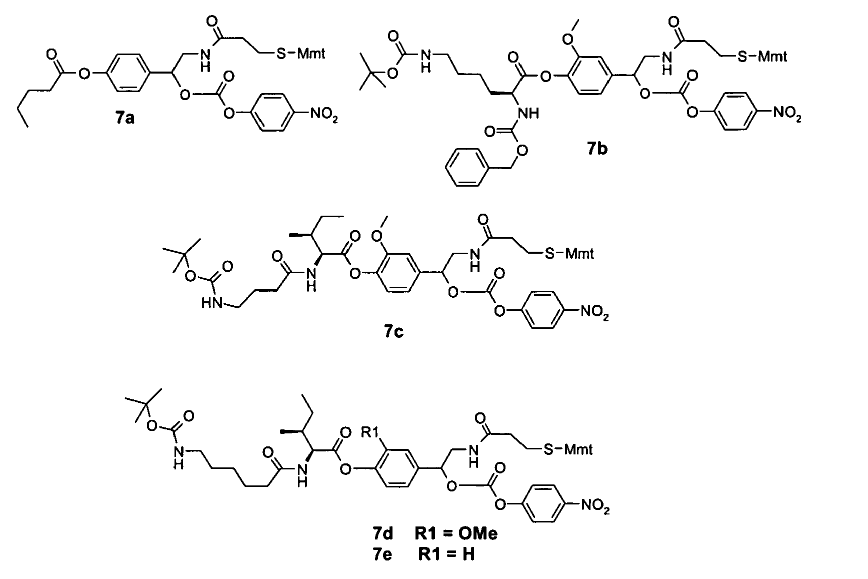

### General synthesis protocol:

Alcohols **6a, 6b, 6c, 6d,** or **6e** (1 eq), 4-nitrophenyl chloroformate (10 eq), and DIEA (10 eq) were stirred in dry dioxane for 3 h at room temperature under nitrogen atmosphere. After addition of acetic acid (25 eq) the mixtures were diluted with 7/3 (v/v) acetonitrile/H₂O and the carbonates **7a, 7b, 7c, 7d,** or **7e,** respectively, were purified by RP-HPLC.

**7a** MS [M+Na]⁺ = 785 (MW+Na calculated = 785.5 g/mol)
**7b** MS [M+Na]⁺ = 1093 (MW+Na calculated = 1093.7 g/mol)
**7c** MS [M+Na]⁺ = 1029 (MW+Na calculated = 1029.6 g/mol)
**7d** MS [M+Na]⁺ = 1057 (MW+Na calculated = 1057.6 g/mol)
**7e** MS [M+Na]⁺ = 1027 (MW+Na calculated = 1027.6 g/mol)

### Synthesis of compounds 8a to 8c (N^{αA1}-linker-insulin)

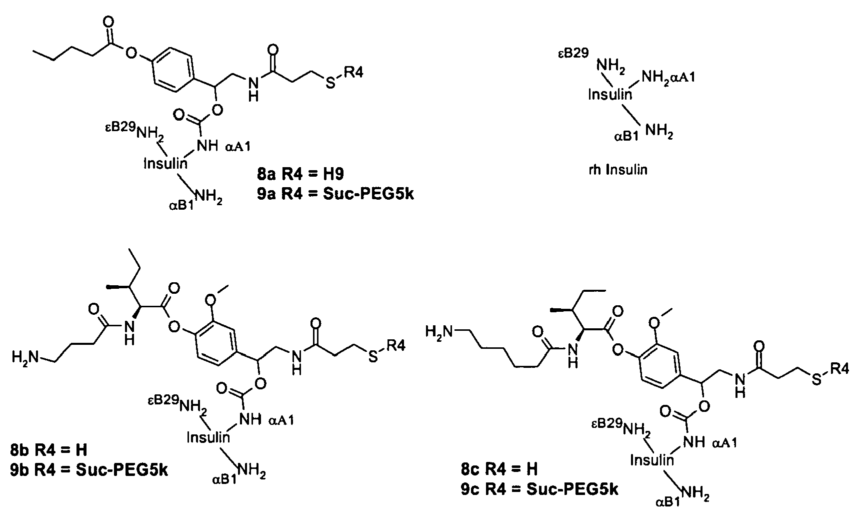

### General synthesis protocol:

Rh-Insulin in 1/1 (v/v) DMSO/DMF was mixed with a solution of 0.9 eq carbonate
**7a, 7c,** or **7d** in DMSO. The resulting solutions were adjusted to basic pH with DIEA and stirred for 1.5 h at RT. RP-HPLC purification gave Mmt-protected intermediates. After lyophilization, the Mmt-protected intermediates were mixed with 95:5 (v/v) TFA/triethylsilane and stirred for 5 min. Volatiles were removed under nitrogen flow and **8a, 8b** or **8c**, respectively, were purified by RP-HPLC and lyophilized. Regioselectivity of insulin modification was verified by DTT reduction and MS analysis.

**8a** MS [M+2H]²⁺ = 3078.9; [M+3H]³⁺ = 2053.2 [M+4H]⁴⁺ = 1540.6 (MW calculated = 6158 g/mol)
**8b** MS [M+2H]²⁺ = 3152.9; [M+3H]³⁺ = 2100.6 [M+4H]⁴⁺ = 1575.8 (MW calculated = 6302 g/mol)
**8c** MS: [M+3H]³⁺ = 2110.7; [M+4H]⁴⁺ = 1583.7; [M+5H]⁵⁺ = 1266.6 (MW calculated = 6330 g/mol)

### Synthesis of compounds 8d to 8g (N^{εB29}-Fluorescein-N^{αA1}-linker-insulin)

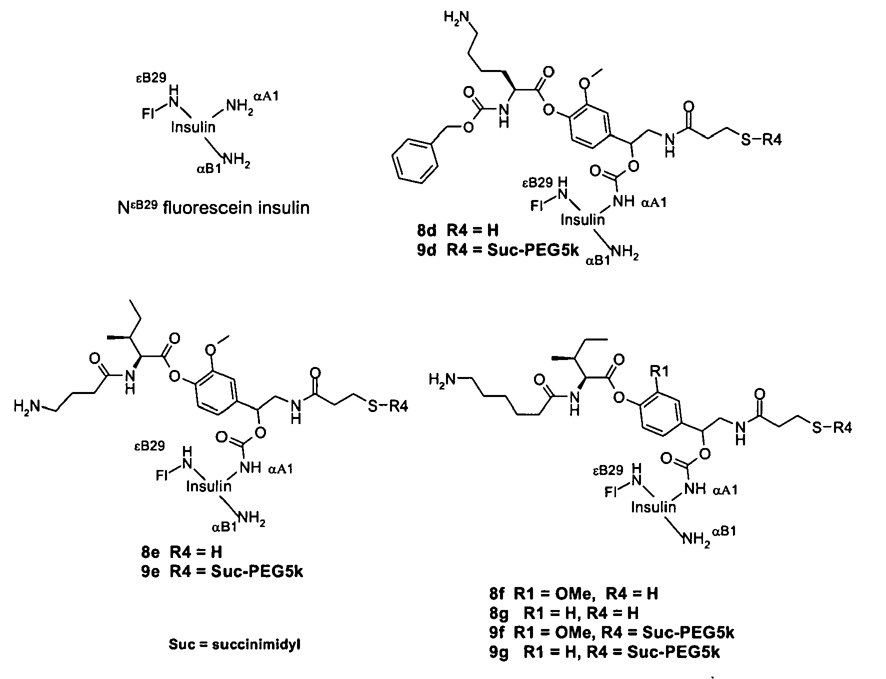

### Synthesis of N^{εB29}-fluorescein insulin:

80 mg (13.8 µmol) rh-insulin were dissolved in 4 ml 1/1 (v/v) DMF/DMSO and 40 µl DIEA were added. 8 mg (17 µmol) 5-(and-6)-carboxyfluorescein succinimidyl ester were added and the solution was stirred for 30 min at room temperature. 4 ml 5/5/1 (v/v/v) acetonitrile/water/acetic acid were added, product N^{εB29}-fluorescein insulin was purified by RP-HPLC and lyophilized. The conjugation site was verified by reduction of N^{εB29}-fluorescein insulin with 1,4-dithiothreitol, protease digestion and MS analysis.

MS: [M+2H]²⁺ = 3084.0; [M+3H]³⁺ = 2054.6 (MW calculated = 6166 g/mol)

### General synthesis protocol:

N^{εB29}-fluorescein insulin in 1/1 (v/v) DMF/DMSO was mixed with a solution of 0.9 eq carbonate **7b, 7c, 7d,** or **7e** in DMSO. The resulting solutions were adjusted to basic pH with DIEA and stirred for 3 h at RT. RP-HPLC purification gave Mmt-protected intermediates.
After lyophilization, the intermediates were dissolved in 95/5 (v/v) TFA/triethylsilane and stirred for 5 min. Volatiles were removed under nitrogen flow and **8d, 8e, 8f,** or **8g** were purified by RP-HPLC and lyophilized.

**8d** MS: [M+2H]²⁺ = 3364.1; [M+3H]³⁺ = 2242.7; [M+4H]⁴⁺ = 1681.5 (MW calculated = 6724 g/mol)
**8e** MS: [M+3H]³⁺ = 2219.2 [M+4H]⁴⁺ = 1665.9; [M+5H]⁵⁺ = 1332.8 (MW calculated = 6660 g/mol)
**8f** MS: [M+3H]³⁺ = 2229.7 [M+4H]⁴⁺ = 1673.3; [M+5H]⁵⁺ = 1337.7 (MW calculated = 6689 g/mol)
**8g** MS: [M+3H]³⁺ = 2218.7 [M+4H]⁴⁺ = 1664.9 (MW calculated = 6659 g/mol)

### Synthesis of compounds 9a to 9g (mono-pegylated insulin compounds)

### General synthesis protocol:

70 µl of a solution of **8a, 8b, 8c, 8d, 8e, 8f,** or **8g** (500 µM) in 1/4 (v/v) acetonitrile/water were mixed with 7 µl of a solution of 10 mM maleimide-PEG5k in 1/4 (v/v) acetonitrile/water and 10 µl of 0.5 M sodium phosphate buffer (pH 7.0) and incubated for 15 min. Compounds **9a, 9b, 9c, 9d, 9e, 9f,** or **9g,** respectively, were purified by SEC (column: Superdex 200, flow rate: 0.75 mL/min) using 10 mM HEPES buffer (pH 7.4), 150 mM NaCl, 3 mM EDTA, and 0.005 % Tween as mobile phase. The collected eluates (approximately 1.5 mL) were directly used as such for release rate determination.

**9a** through **9g**: SEC retention time: 19.5 min

### Synthesis of compounds 11a and 11b

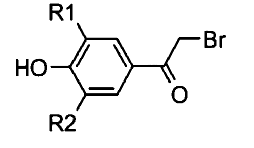

**11a** R1 = R2 = Me
**11b** R1 = Me, R2 = H

### General synthesis protocol:

3,5-Dimethyl-4-hydroxy-acetophenone (5.0 mmol) **10a** or 4-hydroxy-3-methylacetophenone **10b** (0.75 g, 5.0 mmol) and CuBr₂ (1.7 g, 7.5 mmol) were dissolved in 10 ml ethyl acetate and refluxed for 2 h. Solid byproducts were removed by filtration. The filtrate was evaporated and crude products **11a** or **11b** were purified by RP-HPLC.

**11a:** Yield 754 mg (62 %)
MS [M+H]⁺ = 243.1/245.1 (MW+H calculated = 244.1 g/mol)
**11b:** Yield 533 mg (47 %)
MS [M+H]⁺ = 229.2/231.1 (MW+H calculated = 230.1 g/mol)

### Synthesis of compounds 12a and 12b

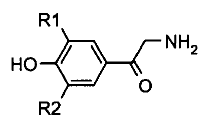

**12a** R1 = R2 = Me
**12b** R1 = Me, R2 = H

### Synthesis protocol:

500 mg **11a** (2.06 mmol) or 472 mg (2.06 mmol) **11b** and 576 mg (4.11 mmol) hexamethylenetetramine were dissolved in 20 ml of trichloromethane and refluxed for 30 min. The solvent was removed in vacuo. 4 ml ethanol and 2 ml of concentrated HCl were added and the slurry was heated to 50 °C for 4 h. The mixture was concentrated in vacuo, diluted with acetonitrile/water and **12a** or **12b** were purified by RP-HPLC.

**12a:** Yield 547 mg (81 %) as TFA-salt
MS [M+Na]⁺ = 202.2 (MW+Na calculated = 202.2 g/mol)
**12b:** Yield 455 mg (70 %) as TFA-salt
MS [M+Na]⁺ = 188.2 (MW+Na calculated = 188,2 g/mol)

### Synthesis of compound 13

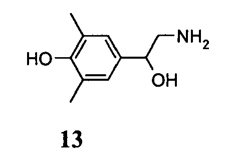

500 mg (1.71 mmol) **12a** (TFA salt) were dissolved in 10 ml 1/1 (v/v) methanol/water, 129 mg (3.41 mmol) NaBH₄ were added and the mixture was stirred for 30 min at RT. 0.5 ml of acetic acid were added and **13** was purified by RP-HPLC.

**13:** Yield 313 mg (62 %) as TFA-salt
MS [M+Na]⁺ = 204.2 (MW+Na calculated = 204.2 g/mol)
NMR (300 MHz, DMSO-d₆) δ[ppm] = 8.25 (s, 1H, Phenol), 7.84 (bs, 3H, NH₃⁺), 6.89 (s, 2H, CHₐᵣ), 5.85 (d, 1H, Hydroxyl, J=3.7 Hz), 4.62 (m, 1H, CH_{Benzyl}), 2.93 (m, 1H, CHₐ), 2.80 (m, 1H, CH_{b}), 2.17 (s, 6H, CH₃).

### Synthesis of compounds 14a to 14c

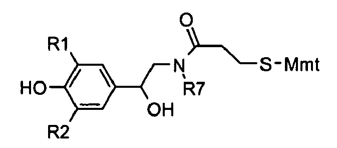

**14a** R1 = R2 = Me, R7 = H
**14b** R1 = R2 = H, R7 = Me
**14c** R1 = OMe, R2 = H, R7 = Me
**13** (TFA salt, 159 mg, 0.541 mmol), synephrine (335 mg, 2.00 mmol), or metanephrine (HCl salt, 281 mg, 1.20 mmol) were coupled to compound **2** as described for compound **3a** to yield **14a, 14b,** or **14c,** respectively.

**14a:** Yield 254 mg (87 %)
MS [M+Na]⁺ = 564.7 (MW+Na calculated = 564.3 g/mol)
**14b:** Yield 760 mg (72 %)
MS [M+Na]⁺ = 550.2 (MW+Na calculated = 550.3 g/mol)
**14c:** Yield 530 mg (80 %)
MS [M+Na]⁺ = 580.4 (MW+Na calculated = 580.4 g/mol)

### Synthesis of compounds 15c, 15d and 15f

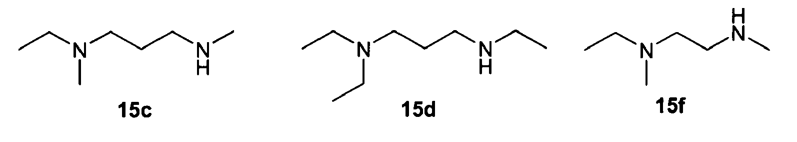

### General synthesis protocol:

1 g 2-chlorotrityl chloride resin (loading 1.4 mmol/g) was incubated for 1 h with N,N'-dimethylpropane-1,3-diamine (for synthesis of **15c**), or N,N'-diethyl-propane-1,3-diamine (for synthesis of **15d**) or N,N'-dimethyl-ethane-1,2-diamine (4 eq) (for synthesis of **15f**) in DCM. After washing of the resin with DMF, amines were acetylated with 1/1/2 (v/v/v) acetic anhydride/pyridine/DMF for 14 h. The resin was washed with THF and dried. LiAlH₄ (1 M in THF, 4 eq) was added dropwise to the suspended resin in THF. The resulting suspension was stirred for 3 h at 45°C under nitrogen atmosphere. After cooling, aqueous Rochelle's salt solution was added and the resin was separated and dried. Compounds were cleaved from resin with 2/1 (v/v) HFIP/DCM (2 x 30 min). The volatile components were evaporated and the products **15c, 15d** or **15f** were used in the following steps without further purification.

**15c** MS [M+H]⁺ = 131.2 (MW = 130.1 g/mol)
**15d** MS [M+H]⁺ = 159.2 (MW = 158.1 g/mol)
**15f** MS [M+H]⁺ = 117.1 (MW = 116 g/mol)

### Synthesis of compounds 16a to 16f

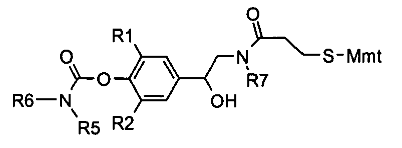

**16a** R1 = R2 = R5 = Me, R6 = 2-(dimethylamino)ethyl, R7 = H
**16b** R1 = OMe, R2 = H, R5 = Et, R6 = 2-(diethylamino)ethyl, R7 = H
**16c** R1 = OMe, R2 = H, R5 = Me, R6 = 3-(N-ethyl-N-methylamino)propyl, R7 = Me **16d** R1 = R2 = H, R5 = Me, R6 = 3-(N-ethyl-N-methylamino)propyl, R7 = Me
**16e** R1 = OMe, R2 = H, R5 = Et, R6 = 3-(diethylamino)propyl, R7 = Me
**16f** R1 = R2 = H, R5 = Et, R6 = 3-(diethylamino)propyl, R7 = Me

**14a** (120 mg, 0.222 mmol) was dissolved in 1.5 ml of dry THF. p-Nitrophenylchloroformate (45 mg, 0.222 mmol) and DIEA (113 µl, 0.665 mmol) were added and the mixture was stirred for 30 min at RT. **15a** (N,N,N'-trimethyl-ethylene-1,2-diamine) (72 µl, 0.554 mmol) was added and stirring was continued for 30 min. The solvent was removed in vacuo, 100 µl of AcOH were added and **16a** was purified by RP-HPLC.

**16b** was synthesized as described above from **3b** (80 mg, 0.15 mmol) and **15b** (N,N,N'-triethyl-ethylene-1,2-diamine) (55 mg, 0.38 mmol).
**16c** or **16d** were synthesized as describe above from **14c** (56 mg, 0.1 mmol) or **14b** (53 mg, 0.1 mmol), respectively, and diamine **15c.**
**16e** or **16f** were synthesized as described above from **14c** (56 mg, 0.1 mmol) or **14b,** respectively, (53 mg, 0.1 mmol) and diamine **15d.**

**16a:** Yield 120 mg (69 %) as TFA salt
MS [M+Na]⁺ = 692.4 (MW+Na calculated = 692.9 g/mol)
**16b:** Yield 48 mg (40 %) as TFA salt
MS [M+Na]⁺ = 736.3 (MW+Na calculated = 736.4 g/mol)
**16c:** Yield 8 mg (10 %) as TFA salt
MS [M+Na]⁺ = 736.4 (MW+Na calculated = 736.4 g/mol)
**16d:** Yield 20 mg (25 %) as TFA salt
MS [M+Na]⁺ = 706.3 (MW+Na calculated = 706.3 g/mol)
**16e:** Yield 2 mg (3 %) as TFA salt
MS [M+Na]⁺ = 764.6 (MW+Na calculated = 764.4 g/mol)
**16f:** Yield 6 mg (8 %) as TFA salt
MS [M+Na]⁺ = 734.4 (MW+Na calculated = 734.3 g/mol)

### Synthesis of compound 17

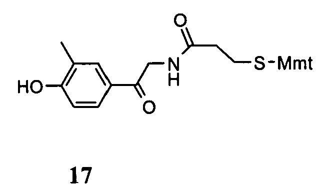

Amine **12b** (TFA salt) was coupled to compound **2** as described for compound **3a**.

**17**: Yield 608 mg (74 %)
MS [M+Na]⁺ = 548.3 (MW+Na calculated = 548.7 g/mol)

### Synthesis of compounds 18a and 18b

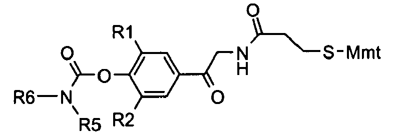

**18a** R1 = R5 = Me, R2 = H, R6 = 3-(dimethylamino)propyl
**18b** R1 = R5 = Me, R2 = H, R6 = 2-(N-ethyl-N-methylamino)ethyl

383 mg (0.729 mmol) of **17** were reacted with p-nitrophenyl-chloroformate and N,N,N'-trimethyl-propane-1,3-diamine (**15e**) or **15f,** respectively, to yield **18a** or **18b** as described for compound **16a.**

**18a:** Yield 287 mg (50 %) as TFA salt
MS [M+Na]⁺ = 690.7 (MW+Na calculated = 690,9 g/mol)
**18b:** Yield 148 mg (26 %) as TFA salt
MS [M+Na]⁺ = 690.9 (MW+Na calculated = 690,9 g/mol)

### Synthesis of compounds 16g and 16h

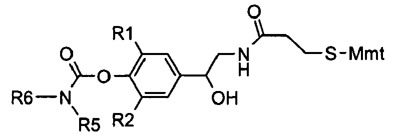

**16g** R1 = R5 = Me, R2 = H, R6 = 3-(dimethylamino)propyl
**16h** R1 = R5 = Me, R2 = H, R6 = 2-(N-ethyl-N-methylamino)ethyl

**18a** (287 mg, 0.367 mmol, TFA salt) was dissolved in 5 ml methanol, NaBH₄ (41 mg, 1.07 mmol) was added and the mixture was stirred for 30 min at RT. 0.5 ml of acetic acid were added and **16g** was purified by RP-HPLC.
**18b** (8 mg, 0.010 mmol, TFA salt) was reacted as described above to yield **16h.**

**16g:** Yield 201 mg (70 %) as TFA-salt
MS [M+Na]⁺ = 692.7 (MW+Na calculated = 692.9 g/mol)
**16h:** Yield 6 mg (77 %) as TFA-salt
MS [M+Na]⁺ = 692.7 (MW+Na calculated = 692.9 g/mol)

### Synthesis of compounds 19a to 19h

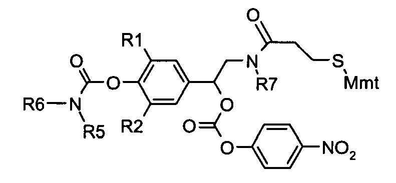

**19a** R1 = R2 = R5 = Me, R6 = 2-(dimethylamino)ethyl, R7 = H
**19b** R 1 = OMe, R2 = H, R5 = Et, R6 = 2-(diethylamino)ethyl, R7 = H
**19c** R1 = OMe, R2 = H, R5 = Me, R6 = 3-(N-ethyl-N-methylamino)propyl, R7 = Me **19d** R1 = R2 = H, R5 = Me, R6 = 3-(N-ethyl-N-methylamino)propyl, R7 = Me
**19e** R1 = OMe, R2 = H, R5 = Et, R6 = 3-(diethylamino)propyl, R7 = Me
**19f** R1 = R2 = H, R5 = Et, R6 = 3-(diethylamino)propyl, R7 = Me
**19g** R1 = R5 = Me, R2 = H, R6 = 3-(dimethylamino)propyl, R7 = H
**19h** R1 = R5 = Me, R2 = H, R6 = 2-(N-ethyl-N-methylamino)ethyl, R7 = H

Carbonates **19a** to **19h** were synthesized from **16a** to **16h,** respectively, as described for compound **7a**.

**19a:** Yield 98 mg (72 %) as TFA-salt
MS [M+Na]⁺ = 857.8 (MW+Na calculated = 858.0 g/mol)
**19b:** Yield 6 mg (11 %) as TFA-salt
MS [M+Na]⁺ = 901.8 (MW+Na calculated = 901.5 g/mol)
**19c:** Yield 1 mg (15 %) as TFA-salt
MS [M+Na]⁺ = 901.4 (MW+Na calculated = 901.5 g/mol)
19d: Yield 8 mg (29 %) as TFA-salt
MS [M+Na]⁺ = 871.4 (MW+Na calculated = 871.4 g/mol)
**19e:** Yield 0.3 mg (18 %) as TFA-salt
MS [M+Na]⁺ = 929.4 (MW+Na calculated = 929.5 g/mol)
**19f:** Yield 4 mg (45 %) as TFA-salt
MS [M+Na]⁺ = 899.7 (MW+Na calculated = 899.6 g/mol)
**19g:** Yield 6 mg (6 %) as TFA-salt
MS [M+Na]⁺ = 857.8 (MW+Na calculated = 858.0 g/mol)
**19h:** Yield 0.8 mg (11 %) as TFA-salt
MS [M+Na]⁺ = 857.7 (MW+Na calculated = 858.0 g/mol)

### Synthesis of compounds 20a to 20f

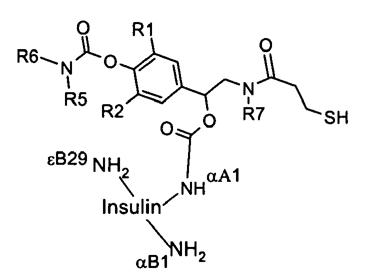

**20a** R1 = R2 = R5 = Me, R6 = 2-(dimethylamino)ethyl, R7 = H
**20b** R1 = OMe, R2 = H, R5 = Et, R6 = 2-(diethylamino)ethyl, R7 = H
**20c** R1 = OMe, R2 = H, R5 = Me, R6 = 3-(N-ethyl-N-methylamino)propyl, R7 = Me
**20d** R1 = R2 = H, R5 = Me, R6 = 3-(N-ethyl-N-methylamino)propyl, R7 = Me
**20e** R1 = R5 = Me, R2 = H, R6 = 3-(dimethylamino)propyl, R7 = H
**20f** R1 = R5 = Me, R2 = H, R6 = 2-(N-ethyl-N-methylamino)ethyl, R7 = H

Insulin derivatives **20a, 20b, 20c, 20d, 20e,** or **20f** were synthesized from **19a, 19b, 19c, 19d, 19g,** or **19h** respectively, as described for compound **8a.**

**20a** MS [M+3H]³⁺ = 2077.3 [M+4H]⁴⁺ = 1559.2 (MW calculated = 6231.3 g/mol)
**20b** MS [M+3H]³⁺ = 2093.0 [M+4H]⁴⁺ = 1569.6 (MW calculated = 6274 g/mol)
**20c** MS [M+3H]³⁺ = 2090.8 [M+4H]⁴⁺ = 1568.7 (MW calculated = 6274 g/mol)
**20d** MS [M+3H]³⁺ = 2081.3 [M+4H]⁴⁺ = 1561.8 (MW calculated = 6244 g/mol)
**20e** MS [M+3H]³⁺ = 2077.1 [M+4H]⁴⁺ = 1558.2 (MW calculated = 6231.3 g/mol)
**20f** MS [M+3H]³⁺ = 2076.7 [M+4H]⁴⁺ = 1559.3 (MW calculated = 6231.3 g/mol)

### Synthesis of compounds 21a to 21f (mono-pegylated insulin derivatives)

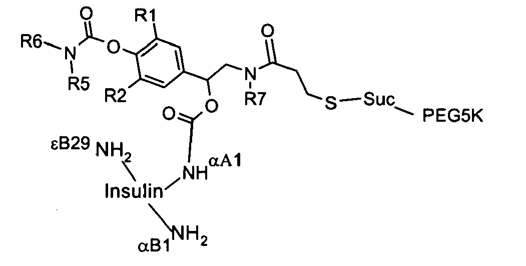

**21a** R1 = R2 = R5 = Me, R6 = 2-(dimethylamino)ethyl, R7 = H
**21b** R1 = OMe, R2 = H, R5 = Et, R6 = 2-(diethylamino)ethyl, R7 = H
**21c** R1 = OMe, R2 = H, R5 = Me, R6 = 3-(N-ethyl-N-methylamino)propyl, R7 = Me
**21d** R1 = R2 = H, R5 = Me, R6 = 3-(N-ethyl-N-methylamino)propyl, R7 = Me
**21e** R1 = R5 = Me, R2 = H, R6 = 3-(dimethylamino)propyl, R7 = H
**21f** R1 = R5 = Me, R2 = H, R6 = 2-(N-ethyl-N-methylamino)ethyl, R7 = H

Insulin derivatives **21a, 21b, 21c, 21d, 21e,** or **21f** were synthesized from compound **20a, 20b, 20c, 20d, 20e,** or **20f,** respectively, as described for compound **9a.**

**21a** through **21f**: SEC retention time: 19.5 min

### Synthesis of compounds 23a and 23b

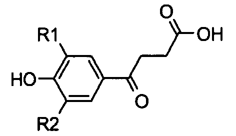

**23a** R1 = Me, R2 = H
**23b** R1 = R2 = Me

o-Cresol (**22a**) or 2,6-dimethylphenol (**22b**) (1 eq), succinic anhydride (1 eq), and AlCl₃ (3 eq) in nitrobenzene were heated to 100 °C for 1 h. The reaction mixture was poured on HCl/ice and extracted with ether. The organic layer was extracted with 1 N NaOH and the aqueous layer was acidified with concentrated HCl. The aqueous layer was extracted with ether and the ether was evaporated. **23a** or **23b** were purified by RP-HPLC.

**23a**: Yield 552 mg (31 %)
MS [M+Na]⁺ = 231.0 (MW+Na calculated = 231.2 g/mol)
NMR (300 MHz, DMSO-d₆) δ[ppm] = 12.05 (bs, 1H, CO₂H), 10.23 (s, 1 H, phenol OH), 7.74 (s, 1H, CHₐᵣ), 7.7 (d, 1H, CHₐᵣ, ³J_{H,H} = 8.4 Hz), 6.86 (d, 1H, CHₐᵣ, ³J_{H,H} = 8.4 Hz), 3.13 (t, 2H, C(O)CH₂, ³J_{H,H} = 6.4 Hz), 2,53 (t, 2H, CH₂CO₂, ³J_{H,H} = 6.4 Hz),
2.16 (s, 3H, CH₃)
**23b:** Yield 166 mg (15 %)
MS [M+Na]⁺ = 245.4 (MW+Na calculated = 245.2 g/mol)

### Synthesis of compound 24

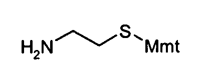

1.85 g (16.02 mmol) cysteamine hydrochloride were dissolved in 15 ml of TFA and 2.47 g (8.01 mmol) MmtCl were added. After stirring the mixture at RT for 20 min the solvent was evaporated in vacuo. The residue was dissolved in diethyl ether and extracted with saturated NaHCO₃, 1N H₂SO₄ and brine. The solvent was evaporated and **24** was purified by RP-HPLC.

**24:** Yield 1.11 g (30 %) as TFA salt
TLC (AcOEt/Et₃N 99/1), R_{f} = 0.24

### Synthesis of compounds 25a and 25b

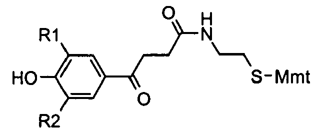

**25a** R1 = Me, R2 = H
**25b** R1 = R2 = Me

**23a** or **23b** (1eq), HOBt (1.1 eq) and DIC (1 eq) were dissolved in DMF and stirred at RT for 30 min. **24** (TFA salt 1 eq) and DIEA (3 eq) were added and the solution was stirred for 60 min. Acetic acid was added and **25a** or **25b** was purified by RP-HPLC.

**25a:** Yield 552 mg (25 %)
MS [M+Na]⁺ = 562.7 (MW+Na calculated = 562.7 g/mol)
**25b:** Yield 15 mg (40 %)
MS [M+Na]⁺ = 576.6 (MW+Na calculated = 576.6 g/mol)

### Synthesis of compounds 26a and 26b

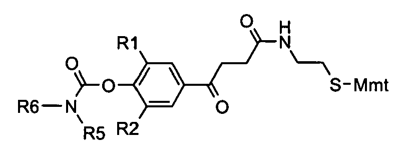

**26a** R1 = Me, R2 = H, R5 = R6 = 3-(dimethylamino)propyl
**26b** R1 = R2 = R5 = Me, R6 = 2-(dimethylamino)ethyl

267 mg (0.495 mmol) **25a** was reacted with p-nitrophenylchloroformate and N-(3-dimethylamino-propyl)-N',N'dimethyl-propane-1,3-diamine (**15g**) to yield **26a** as described for compound **16a.**
**26b** was synthesiszed as described above using 15 mg **25b** and N,N,N'-trimethylethane-1,2-diamine (**15a**).

**26a**: Yield 282 mg (58 %) as double TFA salt
MS [M+Na]⁺ = 775.2 (MW+Na calculated = 776.0 g/mol)
**26b:** Yield 17 mg (70 %) as TFA salt
MS [M+Na]⁺ = 704.5 (MW+Na calculated = 704.6 g/mol)

### Synthesis of compounds 27a and 27b

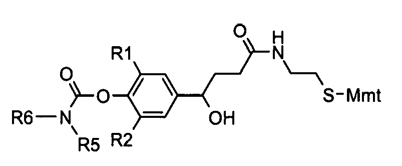

**27a** R1 = Me, R2 = H, R5 = R6 = 3-(dimethylamino)propyl
**27b** R1 = R2 = R5 = Me, R6 = 2-(dimethylamino)ethyl

**26a** (272 mg, 0.277 mmol, double TFA salt) was dissolved in 5 ml methanol, NaBH₄ (42 mg, 1.09 mmol) was added and the mixture was stirred for 30 min at RT. 0.5 ml of acetic acid were added and **27a** was purified by RP-HPLC.
Alcohol **27b** was synthesized likewise from **26b** (17 mg, 25 µmol, TFA salt).

**27a:** Yield 142 mg (52 %) as double TFA salt
MS [M+Na]⁺ = 777.9 (MW+Na calculated = 778.0 g/mol)
**27b:** Yield 6 mg (40 %) as TFA salt
MS [M+Na]⁺ = 706.5 (MW+Na calculated = 706.6 g/mol)

### Synthesis of compounds 28a and 28b

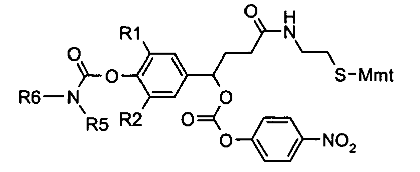

**28a** R1 = Me, R2 = H, R5 = R6 = 3-(dimethylamino)propyl
**28b** R1 = R2 = R5 = Me, R6 = 2-(dimethylamino)ethyl

Carbonates **28a** or **28b** were synthesized from **27a** or **27b**, respectively, as described for compound **7a**.

**28a:** Yield 1 mg (29 %)
MS [M+Na]⁺ = 942.9 (MW+Na calculated = 943.2 g/mol)
**28b:** Yield 1.5 mg (19 %)
MS [M+Na]⁺ = 871.6 (MW+Na calculated = 871.7 g/mol)

### Synthesis of compounds 29a and 29b

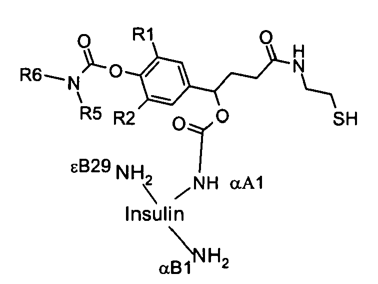

**29a** R1 = Me, R2 = H, R5 = R6 = 3-(dimethylamino)propyl
**29b** R1 = R2 = R5 = Me, R6 = 2-(dimethylamino)ethyl

Insulin derivatives **29a** or **29b** were synthesized from **28a** or **28b**, respectively, as described for compound **8a**.

**29a** MS [M+3H]³⁺ = 2105.8 [M+4H]⁴⁺ = 1580.2 (MW calculated = 6316.4 g/mol)
**29b** MS [M+3H]³⁺ = 2081.8 [M+4H]⁴⁺ = 1562.4 (MW calculated = 6244 g/mol)

### Synthesis of mono-pegylated insulin derivatives 30a and 30b

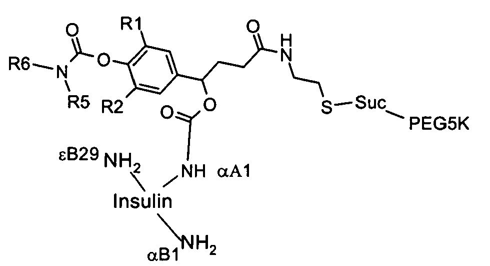

**30a** R1 = Me, R2 = H, R5 = R6 = 3-(dimethylamino)propyl
**30b** R1 = R2 = R5 = Me, R6 = 2-(dimethylamino)ethyl

Insulin derivatives **30a** or **30b** were synthesized from **29a** or **29b**, respectively, as described for compound **9a**.

**30a** and **30b**: SEC retention time: 19.5 min

### Synthesis of a polymeric prodrug according to formula Ia with an ester-linked masking group and a dendritic carrier (9h)

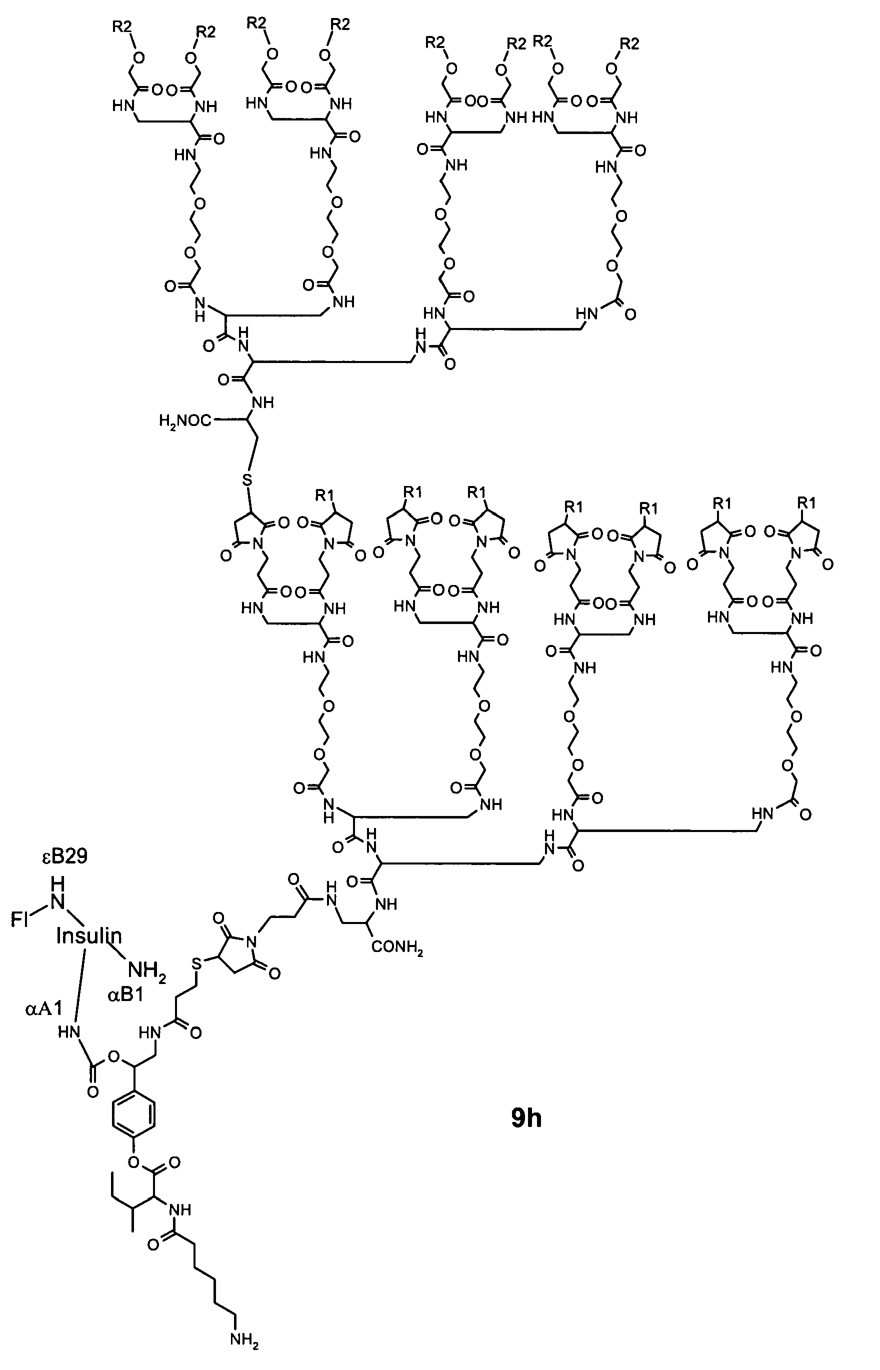

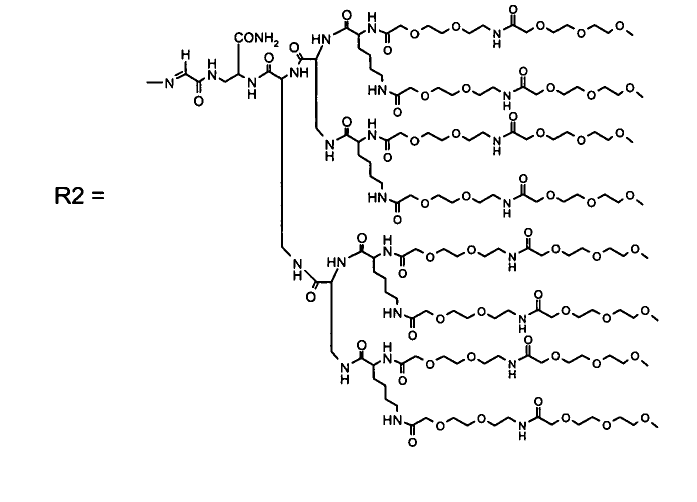

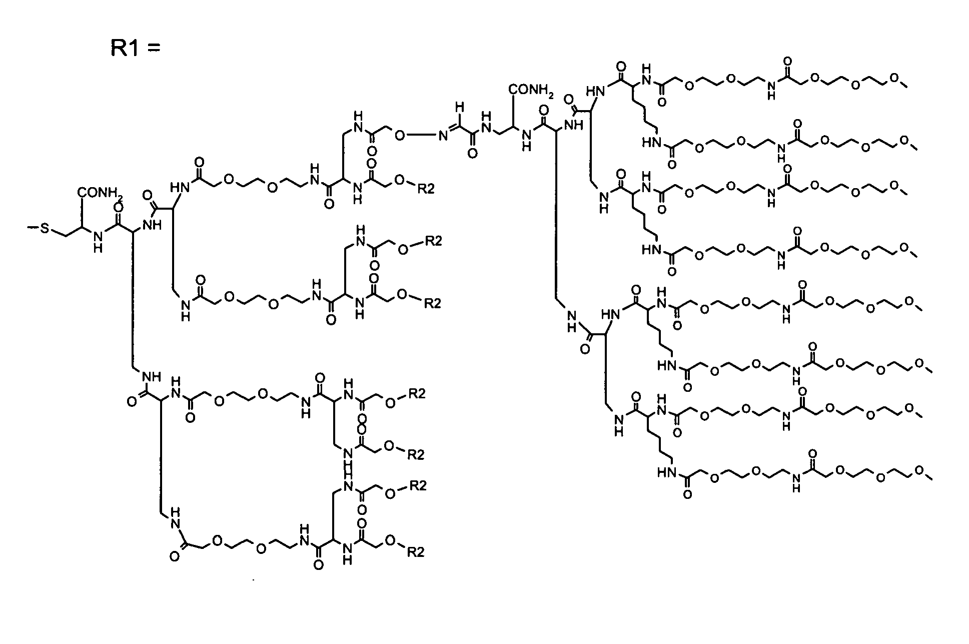

### Synthesis of compound 31

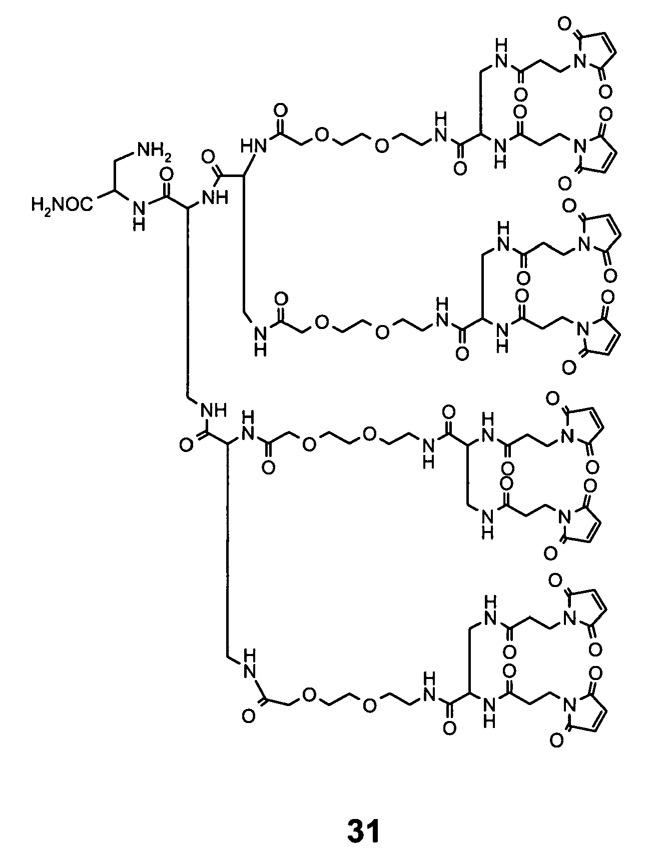

**31** was obtained according to the standard solid-phase synthesis protocol. The amino acids Fmoc-Dpr(Boc)-OH, Fmoc-Dpr(Fmoc)-OH, Fmoc-Dpr(Fmoc)-OH, Fmoc-Ado-OH, and Fmoc-Dpr(Fmoc)-OH were coupled to NovaSyn TG Sieber amide resin. After final fmoc removal the resin was agitated with 5 eq maleimidopropionic acid and 5 eq DIC in relation to amino groups in DMF for 30 min. **31** was cleaved from resin with TFA/TES/water 95/3/2 (v/v/v). After evaporation of solvent, product **31** was purified by RP-HPLC.

MS: [M+H]⁺ = 2494.6 (MW calculated = 2495.4 g/mol)

### Synthesis of compound 32

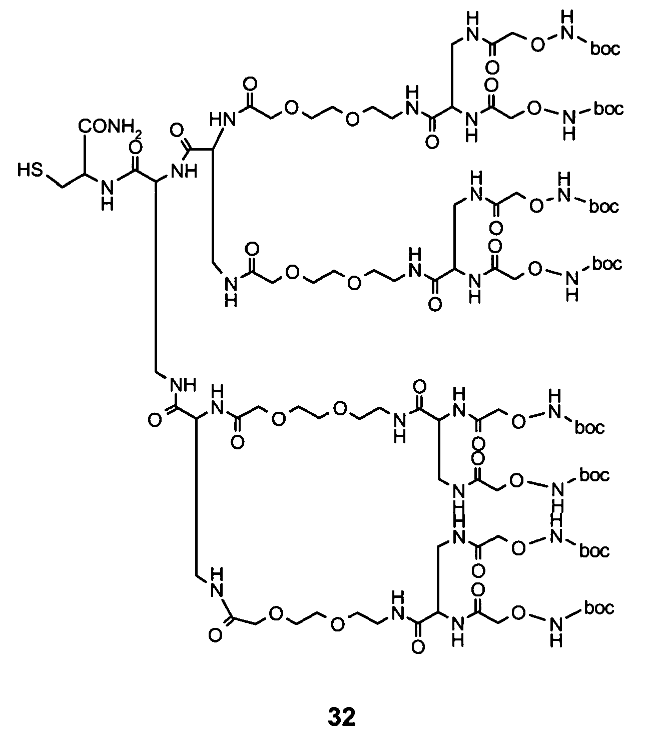

Compound **32** was obtained according to the standard solid-phase synthesis protocol. The amino acids Fmoc-Cys(Mmt)-OH, Fmoc-Dpr(Fmoc)-OH, Fmoc-Dpr(Fmoc)-OH, Fmoc-Ado-OH, and Fmoc-Dpr(Fmoc)-OH were coupled to NovaSynTG Sieber amide resin.
After final fmoc removal the resin was agitated with 3 eq Boc-aminoxyacetic acid, 3 eq DIC, and 3 eq HOBt in relation to amino groups in DMF for 30 min. **32** was cleaved from resin with DCM/TFA/TES 97/1/2 (v/v/v). After addition of 0.8 eq pyridine in relation to TFA the solvent was evaporated and product **32** was purified by RP-HPLC.

MS: [M+H]⁺ = 2688.2 g/mol (MW calculated =2688.8 g/mol)

### Synthesis of compound 33

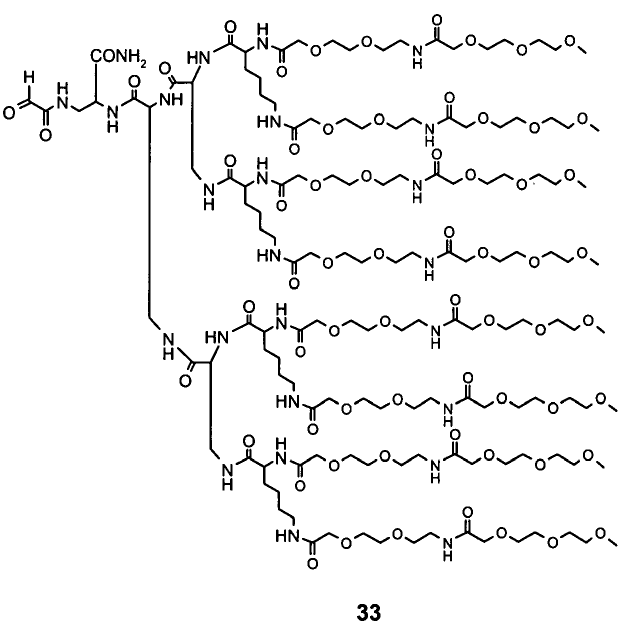

Compound **33** was obtained according to the standard solid-phase synthesis protocol. The amino acids Fmoc-Dpr(ivDde)-OH, Fmoc-Dpr(Fmoc)-OH, Fmoc-Dpr(Fmoc)-OH, Fmoc-Lys(Fmoc)-OH, and Fmoc-Ado-OH were coupled to NovaSyn TG Sieber amide resin.
After final fmoc removal the resin was agitated with 3 eq 3,6,9-trioxadecanoic acid, 3 eq PyBOP, and 6 eq DIEA in relation to amino groups in DMF for 60 min.
To cleave the ivDde protecting group, the resin was treated three times with 2 % hydrazine in DMF. After washing, 3 eq Fmoc-Ser-OH was coupled with 3eq DIC and 3 eq HOBt for 30 min. After final fmoc removal resin was washed and the product was cleaved from resin with DCM/TFA/TES 88/10/2 (v/v/v). Solvent was evaporated and the residue was oxidized with 10 eq sodium periodate in 3/2 (v/v) 0.1 M sodium phosphate pH 7/acetonitrile for 15 min to yield 3**3.** Product **33** was purified by RP-HPLC and lyophilized.

MS: [M+H]⁺ = 3372.1 g/mol (3372.8 g/mol)

### Synthesis of compound 34

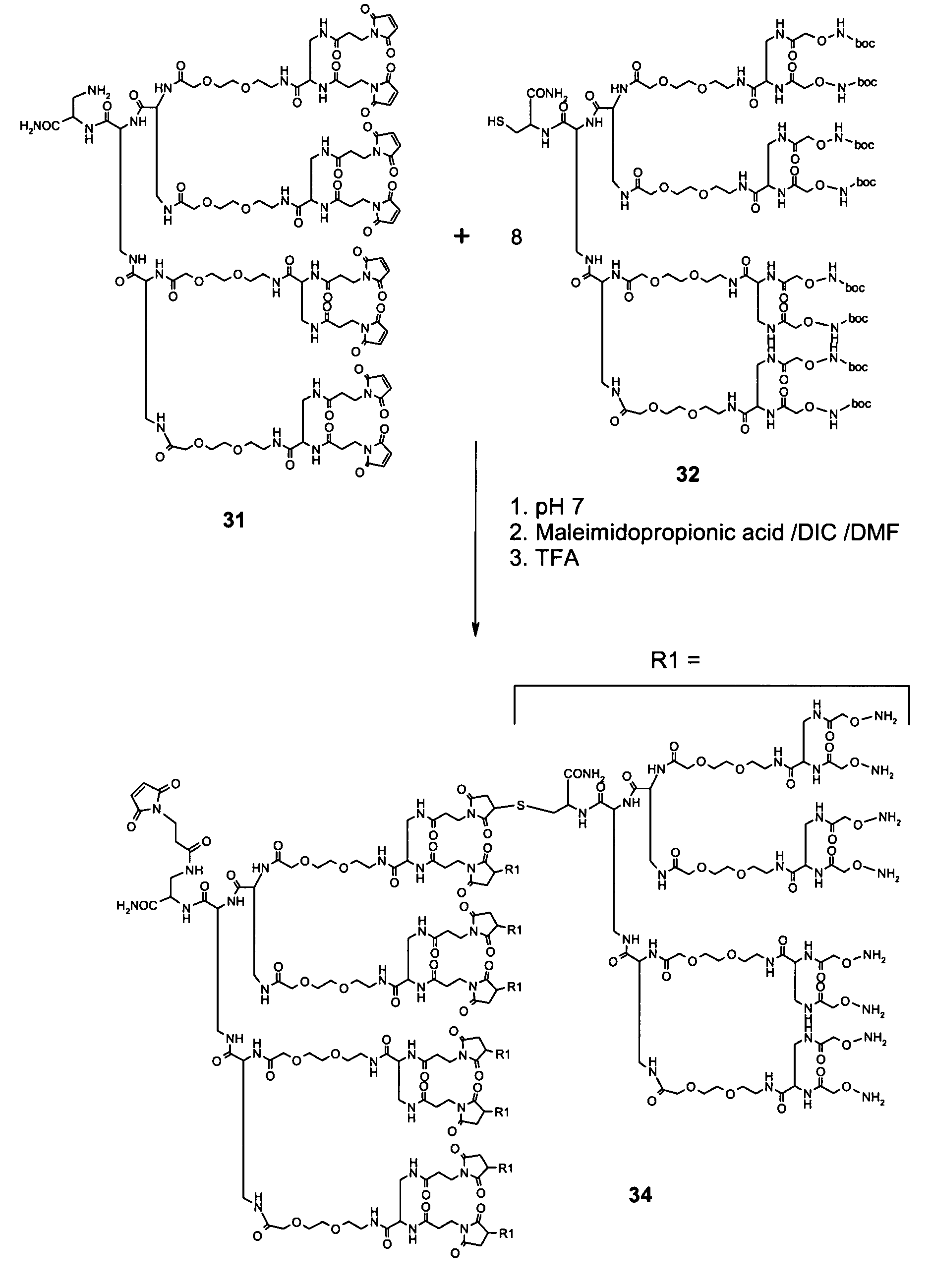

6 mg (2.4 µmol) of compound **31** were dissolved in 1 ml 2/1 (v/v) acetonitrile/0.1 M sodium phosphate buffer pH 7 and 65 mg (24.2 µmol) of compound **32** were added. The solution was stirred at room temperature for 2 h and then the product purified by RP-HPLC and lyophilized (yield: 45 mg (78 %)).

The lyophilized product (45 mg) was dissolved in 0.5 ml DMF and 10 µl DIEA were added. 5 mg (30µmol) 3-maleimidopropionic acid and 4.7 µl (30 µmol) DIC in 150 µl DMF were added and the reaction mixture was stirred at room temperature for 20 min, the product purified by RP-HPLC and lyophilized.

The lyophilized product was incubated for 10 min in 95/5 (v/v) TFA/water and then the solvent was removed in a stream of nitrogen. Product **34** was purified by RP-HPLC and lyophilized (overall yield for all three steps: 20 mg (47 %)).

### MS: 17700 - 18200 (broad peak) (MW calculated = 17749 g/mol)

### Synthesis of compound 9h

1.5 mg (225 nmol) **8g** and 5 mg (280 nmol) 34 were mixed, dissolved in 300 µl 2/1 (v/v) 0.1 M sodium phosphate buffer pH 7/acetonitrile and incubated for 15 min at room temperature. The product was purified by RP-HPLC and lyophilized. (yield 4 mg, 160 nmol, 70 %)

The lyophilized product was dissolved in 200 µl 0.1 M sodium citrate buffer pH 1.5 and 69 mg (20.5 µmol) **33** in 200 µl 2/1 (v/v) acetonitrile/sodium citrate buffer pH 1.5 were added. The mixture was stirred at room temperature for 24 h and product **9h** was purified by size exclusion chromatography (column: Superdex 200, buffer: 10 mM HEPES pH 7.4, 0.005 % Tween-20, 3mM EDTA, flow rate: 0.75 ml/min)

SEC elution time: 15 min

### Synthesis of compound 37a

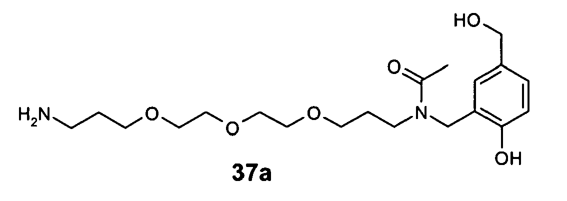

250 mg (0.35 mmol) 2-chlorotrityl chloride resin (loading 1.4 mmol/g) was incubated for 1.5 h with 308 mg (4 eq., 1.4 mmol) 4,7,10-trioxatridecane-1,13-diamine in 4 ml DCM to yield **35a**. The resin was washed with DCM and dried. 107 mg (0.7 mmol) HOBt, 110 µl (0.7 mmol) DIC, and 150 mg (0.9 mmol) 5-formyl salicylic acid in 3 ml DMF were added and the resulting suspension was stirred for 1 h at RT to yield **36a**. After washing with DCM and THF, the resin was suspended in 6 mL THF and 3 ml (3 mmol) BH₃ THF (1 M in THF, 8.5 eq.) were added dropwise. The reaction mixture was stirred for 18 h at 45°C under nitrogen atmosphere. After cooling 4 ml THF, 0.8 ml DIEA and 1.6 ml MeOH were added successively. 210 mg (0.84 mmol) I₂ (as a concentrated THF solution) were added and the suspension was stirred for 1 h. The resin was repeatedly washed (three times each) with THF, DMF, MeOH, and DCM. The dried resin was reacted with 107 mg (0.7 mmol) HOBt, 110 µl (0.7 mmol) DIC, and 55 µL (0.9 mmol) AcOH in 3 ml DMF for 1h. After washing of the resin with DMF and DCM compound **37a** was cleaved from resin with 2/1 (v/v) HFIP/DCM (two times for 30 min). The volatile components were evaporated and the product **37a** was used in the following step without further purification.

**37a:** Yield 29 mg (20 %) as TFA salt
MS [M+Na]⁺ = 421.4 (MW+Na calculated = 421.5 g/mol)

### Synthesis of compound 38

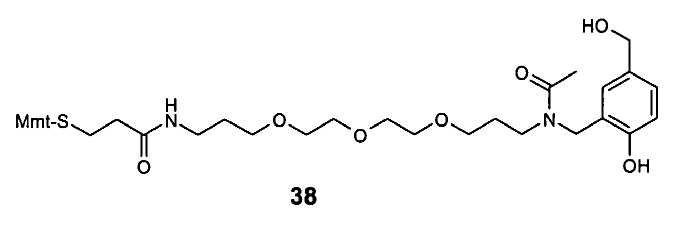

24 mg (0.06 mmol) **37a,** 31 mg (0.06 mmol) PyBOP, 32 µl (0.18 mmol) DIEA, and 23 mg (0.06 mmol) **2** in 0.5 mL DMF were reacted for 50 min at room temperature. After addition of 50 µl acetic acid product **38** was purified by RP-HPLC.

**38**: Yield 7 mg (15 %)
MS [M+Na]⁺ = 781.3 (MW+Na calculated = 781.6 g/mol)

### Synthesis of compound 39

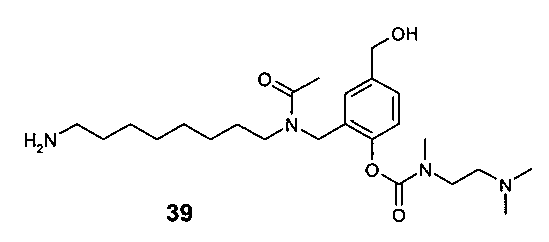

300 mg (0.42 mmol) 2-chlorotrityl chloride resin (loading 1.4 mmol/g) was incubated for 1.5 h with 245 mg (4 eq., 1.7 mmol) 1,8-diaminooctane in 4 ml DCM to yield **35b.** The resin was washed with DCM and dried. 107 mg (0.7 mmol) HOBT, 110 µl (0.7 mmol) DIC, and 150 mg (0.9 mmol) 5-formyl salicylic acid in 3 ml DMF were added and the resulting suspension was stirred for 1 h at RT to yield **36b.** After washing with DCM and THF, the resin was suspended in 6 mL THF and 3 ml (3 mmol) BH₃ THF (1 M in THF) were added dropwise. The reaction mixture was stirred for 18 h at 45°C under nitrogen atmosphere. After cooling 4 ml THF, 0.8 ml DIEA and 1.6 ml MeOH were added successively. 210 mg (0.84 mmol) I₂ (as a concentrated THF solution) were added and the suspension was stirred for 1 h. The resin was repeatedly washed (three times each) with THF, DMF, MeOH, and DCM. The dried resin was reacted with 107 mg (0.7 mmol) HOBT, 110 µl (0.7 mmol) DIC, and 55 µL (0.9 mmol) AcOH in 3 ml DMF for 1h. After washing of the resin with DMF and DCM compound **37b**, 78 mg (0.39 mmol) p-nitrophenylchloroformate, and 210 µl (1.2 mmol) DIEA in 1/1 (v/v) THF/DCM were reacted for 30 min at RT. The separated resin was suspended in 1/1 (v/v) THF/DCM and 210 µl (1.2 mmol) N,N,N'-trimethylethylendiamine were added. The resulting suspension was stirred for 25 min at RT. The resin was separated and washed with DCM. Product 39 was cleaved from resin with 2/1 (v/v) HFIP/DCM (two times for 30 min). The volatile components were evaporated and the product **39** was purified by HPLC.

**39**: Yield 16 mg (8 %) as TFA salt
MS [M+Na]⁺ = 473.5 (MW+Na calculated = 473.3 g/mol)

### Synthesis of compound 40a

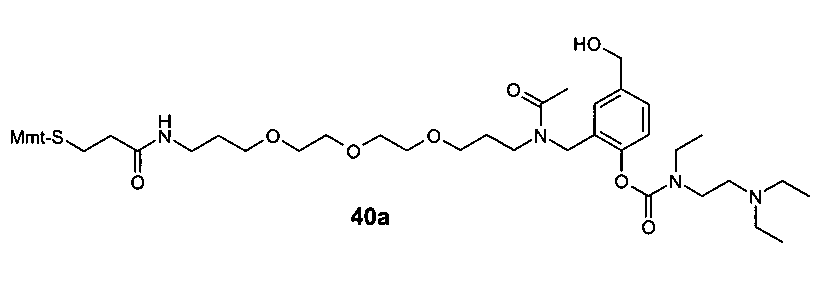

**38** (7 mg, 9 µmol) was dissolved in 200 µl of dry THF. p-Nitrophenylchloroformate (2.0 mg, 10µmol) and DIEA (4.4 µl, 25 µmol) were added and the mixture was stirred for 30 min at RT. N,N,N'-Triethylethylenediamine (**15b**) (18 µl, 0.1 mmol) was added and stirring was continued for 30 min. The solvent was removed *in vacuo,* 10 µl of AcOH were added and **40a** was purified by RP-HPLC.

**40a:** Yield 1 mg (11 %) as TFA salt
MS [M+Na]⁺ = 951.1 (MW+Na calculated = 951.8 g/mol)

### Synthesis of compound 40b

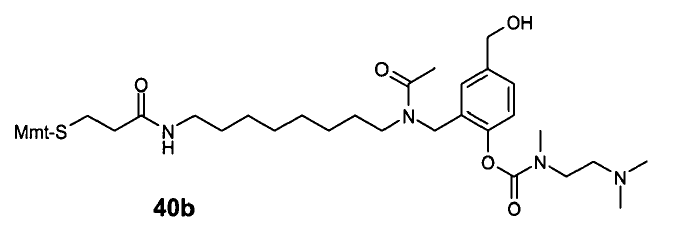

15 mg (33 µmol) **39,** 18 mg (33 µmol) PyBOP, 23 µl (0.13 mmol) DIEA, and 13 mg (35 µmol) **2** in 0.5 mL DMF were reacted for 45 min at room temperature. After addition of 50 µl acetic acid product **40** was purified by RP-HPLC.

**40b**: Yield 10 mg (37 %) as TFA salt
MS [M+H]⁺ = 811.5 (MW+Na calculated = 810.5 g/mol)

### Synthesis of compound 41a and 41b

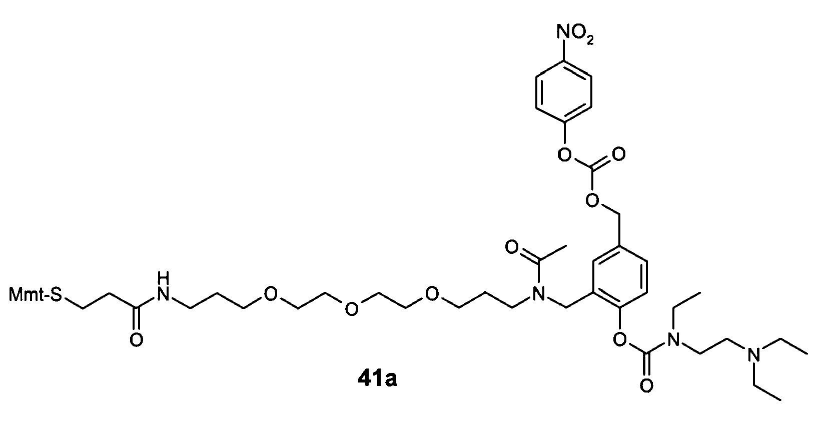

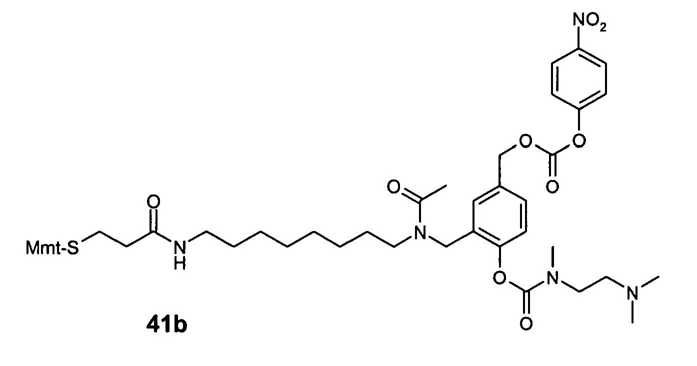

Carbonate **41a** or **41b** was synthesized from **40a** or **40b** as described for compound **7a**

**41a:** Yield 0.4 mg as TFA salt
MS [M+Na]⁺ = 1116.8 (MW+Na calculated = 1116.9 g/mol)
**41b:** Yield 2 mg (16 %) as TFA salt
MS [M+H]⁺ = 976.8 (MW calculated = 975.8 g/mol)

### Synthesis of compound 42

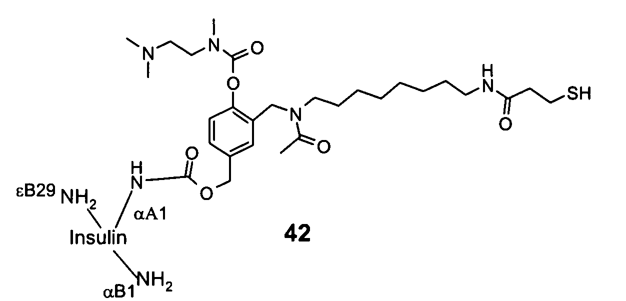

Insulin derivative **42** was synthesized from **41b** as described for compound **8a**.

**42** MS [M+3H]³⁺ = 2124.5 [M+4H]⁴⁺ = 1594.6 (MW calculated = 6371 g/mol)

### Synthesis of compound 43

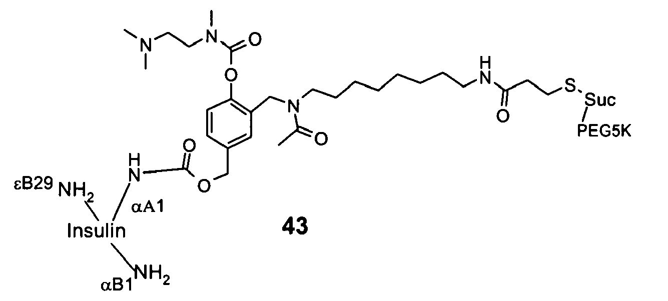

Insulin derivative **43** was synthesized from **42** as described for compound **9a**.

**43**: SEC retention time: 18.0 min

### Synthesis of rh-insulin loaded PEGA hydrogel 45

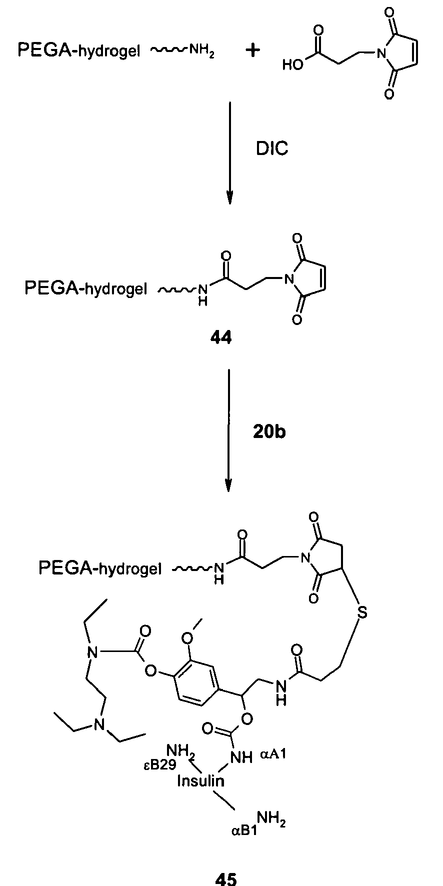

### Maleimide derivatization of polyacrylamide based hydrogel (PEGA):

NH₂-PEGA hydrogel beads with 0.4 mmol/g loading and 150-300 µm bead size were purchased from Novabiochem.
2.5 g methanol-wet NH₂-PEGA-hydrogel (0.4 mmol/g NH₂-loading) was weighed into a syringe equipped with a polypropylene frit. Maleimide loading was adjusted by acylation employing a mixture of activated maleimidopropionic acid and acetic acid as described in the following. The hydrogel was washed 5 times with DMF and reacted with 13.5 mg (0.08 mmol) 3-maleimidopropionic acid, 115.2 µl (1.92 mmol) acetic acid and 313 µl (2 mmol) DIC in 4 ml DMF for 30 min. The maleimide derivatized hydrogel **44** was washed 10 times with DMF and DCM and finally with acetonitrile.

30 mg of maleimide derivatized resin **44** (loading 16 µmol/g) was reacted with 3 mg of compound **20b** (480 nmol, 1.06 eq) in 600 µl 20/80 (v/v) acetonitrile/50 mM phosphate buffer (pH 7.4) for 10 min to give rh-insulin loaded hydrogel **45**. The hydrogel **45** was washed 5 times with 50/50 (v/v) acetonitrile/water and three times with acetonitrile and dried under vacuum.

### Synthesis of rh-insulin carbohydrate-based hydrogel 46

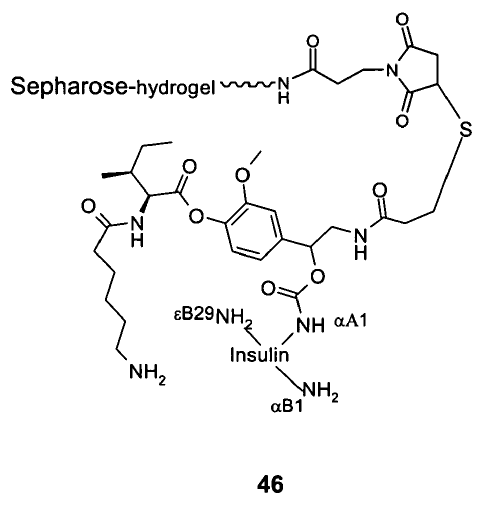

NHS-activated "Sepharose 4 Fast Flow" hydrogel beads (chemically crosslinked agarose, crosslinker epichlorhydrin) were purchased from Amersham.
1.5 g ethanol-wet Sepharose hydrogel (150 mg dry hydrogel) was weighed into a syringe equipped with a polypropylene frit and reacted with 1 M 4,7,10-trioxatridecane-1,13-diamine in DMF for 30 min. After 5 washing steps with DMF, hydrogel was reacted with 8.5 mg (0.05 mmol) 3-maleimidopropionic acid, 57 µl (0.95 mmol) acetic acid, 151 mg (1 mmol) HOBt and 158 µl (1 mmol) DIC in 4 ml DMF for 30 min to give maleimide derivatized hydrogel. The hydrogel was washed 10 times with DMF and finally with acetonitrile.

1.5 mg **8c** was dissolved in 25/75 (v/v) acetonitrile/50 mM phosphate buffer pH 7.4 and reacted with 10.8 mg maleimide derivatized hydrogel for 10 min. The rh-insulin loaded hydrogel **46** was washed five times with 50/50 (v/v) acetonitrile/water and three times with acetonitrile and dried under vacuum.

### Release of insulin or fluorescein-insulin from conjugates in buffer pH 7.4

Release of (fluorescein)-insulin from (fluorescein)-insulin conjugates **9a** to **9h**, **21a** to **21f**, **30a, 30b,** and **43** was effected by linker hydrolysis in aqueous buffer pH 7.4. Collected SEC eluates of (fluorescein) insulin conjugates (see above) were incubated at 37 °C and samples were taken at time intervals and analyzed by RP-HPLC (insulin conjugates) or SEC (fluorescein insulin conjugates) and UV detection. Peaks correlating with the retention time of native insulin or fluorescein-insulin, respectively, were integrated and plotted against incubation time, and curve-fitting software was applied to estimate the corresponding halftime of release.

### Release of insulin from hydrogel conjugates 45 and 46

4 mg of **45** or 2 mg **46** were weighed into a test tube and incubated with 1 ml 10 mM HEPES buffer pH 7.4, 150 mM NaCl, 0.005 % Tween at 37°C. 45 µl samples were taken at different time intervals and quantitatively analyzed for rh-insulin by a RP-HPLC assay. The rh-insulin peak was integrated and rh-insulin concentration was obtained from a standard curve. A first order release kinetic was fitted to the data points to give the linker half life.

### MS-Analysis of released insulin from compound 9a, 9b and 30a

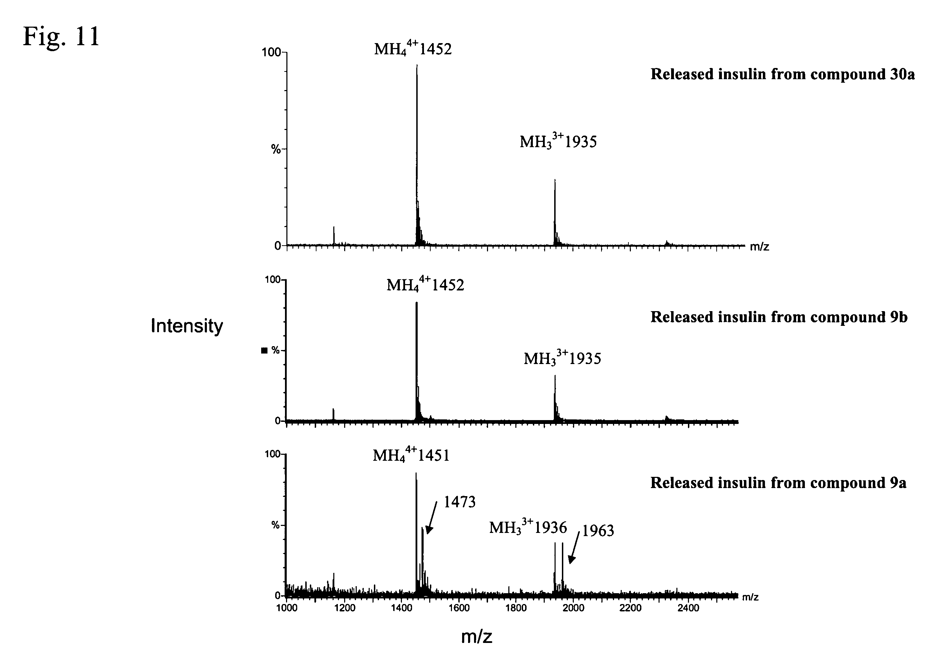

Samples of buffer released insulin (see above) were analyzed by mass spectrometry. Figure 11 shows the mass spectra of released insulin from compound **9a, 9b,** and **30a**. The mass spectrum of insulin released from compound **9a** clearly shows a major side product (indicated by arrows), corresponding to the irreversibly pentanoyl-modified insulin. In this case, removal of the pentanoyl masking goup was not by hydrolysis but by acyl transfer to the insulin. The mass spectrum of insulin released from compound **9b** and **30a** shows no modification.

### Release of fluorescein-insulin from conjugate 9d and 9e in 80 % human plasma

Release of fluorescein-insulin from **9d** or **9e** was effected by hydrolysis in 80 % human plasma in 20 mM HEPES pH 7.4 at 37 °C. Samples were taken at time intervals and analyzed by SEC and UV detection. Peaks correlating with the retention time of fluorescein-insulin were integrated and plotted against incubation time, and curve-fitting software was applied to estimate the corresponding halftime of release.

**Table: Polymeric prodrug hydrolysis**

| compound | t_{1/2} buffer pH 7.4 | t_{1/2} human plasma |
|---|---|---|
| **9a** | 40 h | nd |
| **9b** | 55 h | nd |
| **9c** | 4.5 d | nd |
| **9d** | 7 h | 4 h |
| **9e** | 55 h | 30 h |
| **9f** | 90 h | nd |
| **9g** | 37 h | nd |
| **9h** | 88 h | nd |
| **21a** | 64 d | nd |
| **21b** | 8 d | nd |
| **21c** | 52 d | nd |
| **21d** | 29 d | nd |
| **21e** | 100 d | nd |
| **21f** | 83 h | nd |
| **30a** | 17 d | nd |
| **30b** | > 70 d | nd |
| **45** | 7 d | nd |
| **46** | 4 d | nd |
| **43** | 4 h | nd |

| | | |
|---|---|---|
| nd = not determined | | |

Abbreviations:
- Boc: t-butyloxycarbonyl
- DBU: 1,3-diazabicyclo[5.4.0]undecene
- DCM: dichloromethane
- (iv)Dde: 1-(4,4-dimethyl-2,6-dioxo-cyclohexyliden)3-methyl-butyl
- DIC: diisopropylcarbodiimide
- DIEA: diisopropylethylamine
- DMAP: dimethylamino-pyridine
- DMF: N,N-dimethylformamide
- DMSO: dimethylsulfoxide
- Dpr: diaminopropionic acid
- EDTA: ethylenediaminetetraacetic acid
- Et: ethyl
- eq: stoichiometric equivalent
- fmoc: 9-fluorenylmethoxycarbonyl
- Fmoc-Ado-OH: Fmoc-8-amino-3,6-dioxaoctanoic acid
- HFIP: hexafluoroisopropanol
- HEPES: N-(2-hydroxyethyl) piperazine-N'-(2-ethanesulfonic acid)
- HOBt: N-hydroxybenzotriazole
- LCMS: mass spectrometry-coupled liquid chromatography
- Mal: maleimidopropionyl
- Me: methyl
- Mmt: 4-methoxytrityl
- MS: mass spectrum
- MW: molecular mass
- PyBOP: benzotriazole-1-yl-oxy-tris-pyrrolidino-phosphonium hexafluorophosphate
- RP-HPLC: reversed-phase high performance liquid chromatography
- RT: room temperature
- SEC: size exclusion chromatography
- Suc: succinimidopropionyl
- TES: triethylsilane
- TFA: trifluoroacetic acid
- THF: tetrahydrofurane
- UV: ultraviolet
- Z: benzyloxycarbonyl

## Claims

1. A molecule having the following structure: 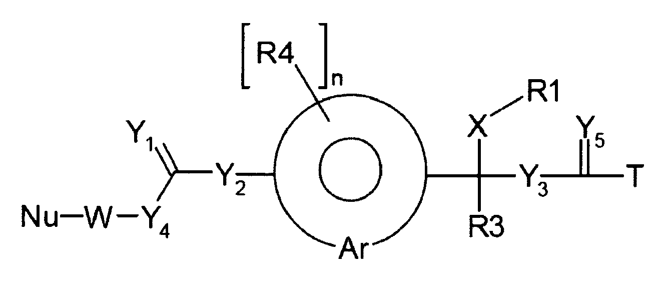 wherein
T is D or A
D being a residue of an amine containing biologically active moiety and
A being a leaving group;
X is a spacer moiety such as R5-Y₆
Y₁, Y₂ can each be either O, S, or NR6, independently of each other.
Y₃, Y₅ can each be either O or S, independently of each other.
Y₄ is O, NR6, or -C(R7)(R8)-
Y₆ is O, S, NR6, succinimide, maleimide, unsaturated carbon-carbon bonds or any heteratom containing a free electron pair or is absent.
R3 is selected from hydrogen, substituted or non-substituted linear, branched or cyclical alkyl or heteroalkyl, aryls, substituted aryls, substituted or non-substituted heteroaryls, cyano, nitro, halogen, carboxy, carboxyalkyl, alkylcarbonyl, or carboxamidoalkyl;
R4 is selected independently from hydrogen, substituted or non-substituted linear, branched or cyclical alkyl or heteroalkyl, aryl, substituted aryl, substituted or non-substituted heteroaryl, substituted or non-substituted linear, branched, or cyclical alkoxy, substituted or non-substituted linear, branched, or cyclical heteroalkyloxy, aryloxy, or heteroaryloxy;
R5 is selected from substituted or non-substituted linear, branched or cyclical alkyl or heteroalkyl, aryls, substituted aryls, substituted or non-substituted heteroaryls, carboxyalkyl, alkylcarbonyl or carboxamidoalkyl;
R7 and R8 are selected from hydrogen, substituted or non-substituted linear, branched or cyclical alkyl or heteroalkyl, aryls, substituted aryls, substituted or non-substituted heteroaryls, carboxyalkyl, alkylcarbonyl, carboxamidoalkyl, cyano, or halogen;
R6 is selected from hydrogen, substituted or non-substituted linear, branched or cyclical alkyl or heteroalkyl, aryls, substituted aryls, substituted or non-substituted heteroaryls;
R1 is a polymer;
W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl; Nu is a nucleophile;
n is zero or a positive integer; and
Ar is a multi-substituted aromatic hydrocarbon or a multi-substituted aromatic heterocycle.

2. A molecule having the following structure: 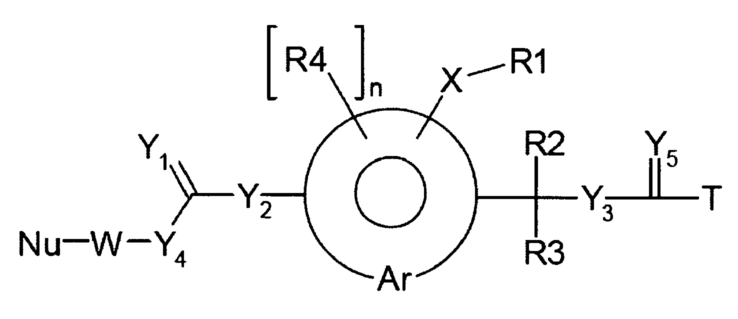 wherein
T is D or A
D being a residue of an amine containing biologically active molecule and
A being a leaving group;
X is a spacer moiety such as R5-Y₆
Y₁, Y₂ can each be either O, S, or NR6, independently of each other.
Y₃, Y₅ can each be either O or S, independently of each other.
Y₄ is O, NR6, or -C(R7)(R8)-
Y₆ is O, S, NR6, succinimide, maleimide, unsaturated carbon-carbon bonds or any heteratom containing a free electron pair or is absent
R2 and R3 are selected independently from hydrogen, substituted or non-substituted linear, branched or cyclical alkyl or heteroalkyl, aryls, substituted aryls, substituted or non-substituted heteroaryls, cyano, nitro, halogen, carboxy, carboxyalkyl, alkylcarbonyl, or carboxamidoalkyl;
R4 is selected independently from hydrogen, substituted or non-substituted linear, branched or cyclical alkyl or heteroalkyl, aryl, substituted aryl, substituted or non-substituted heteroaryl, substituted or non-substituted linear, branched, or cyclical alkoxy, substituted or non-substituted linear, branched, or cyclical heteroalkyloxy, aryloxy, or heteroaryloxy;
R5 is selected from substituted or non-substituted linear, branched or cyclical alkyl or heteroalkyl, aryls, substituted aryls, substituted or non-substituted heteroaryls, carboxyalkyl, alkylcarbonyl or carboxamidoalkyl;
R7 and R8 are selected from hydrogen, substituted or non-substituted linear, branched or cyclical alkyl or heteroalkyl, aryls, substituted aryls, substituted or non-substituted heteroaryls, carboxyalkyl, alkylcarbonyl, carboxamidoalkyl, cyano, or halogen;
R6 is selected from hydrogen, substituted or non-substituted linear, branched or cyclical alkyl or heteroalkyl, aryls, substituted aryls, substituted or non-substituted heteroaryls;
R1 is a polymer;
W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl; Nu is a nucleophile;
n is zero or a positive integer; and
Ar is a multi-substituted aromatic hydrocarbon or a multi-substituted aromatic heterocycle.

3. The molecule of claim 1 or 2, wherein the biologically active moiety is selected from the group of biologically moieties consisting of small molecule biologically active agents or biopolymers.

4. The molecule of claim 3, wherein the biopolymers are selected from the group of biopolymers consisting of proteins, polypeptides, oligonucleotides and peptide nucleic acids.

5. The molecule of claim 4, wherein the polypeptides are selected from the group of polypeptides consisting of ACTH, adenosine deaminase, agalsidase, albumin, alfa-1 antitrypsin (AAT), alfa-1 proteinase inhibitor (API), alteplase, anistreplase, ancrod serine protease, antibodies (monoclonal or polyclonal, and fragments or fusions), antithrombin III, antitrypsins, aprotinin, asparaginases, biphalin, bone-morphogenic proteins, calcitonin (salmon), collagenase, DNase, endorphins, enfuvirtide, enkephalins, erythropoietins, factor VIIa, factor VIII, factor VIIIa, factor IX, fibrinolysin, fusion proteins, follicle-stimulating hormones, granulocyte colony stimulating factor (G-CSF), galactosidase, glucagon, glucocerebrosidase, granulocyte macrophage colony stimulating factor (GM-CSF), phospholipase-activating protein (PLAP), gonadotropin chorionic (hCG), hemoglobins, hepatitis B vaccines, hirudin, hyaluronidases, idurnonidase, immune globulins, influenza vaccines, interleukins (1 alfa, 1 beta, 2, 3, 4, 6, 10, 11, 12), IL-1 receptor antagonist (rhIL-1ra), insulins, interferons (alfa 2a, alfa 2b, alfa 2c, beta 1a, beta 1b, gamma 1a, gamma 1b), keratinocyte growth factor (KGF), transforming growth factors, lactase, leuprolide, levothyroxine, luteinizing hormone, lyme vaccine, natriuretic peptide, pancrelipase, papain, parathyroid hormone, PDGF, pepsin, platelet activating factor acetylhydrolase (PAF-AH), prolactin, protein C, octreotide, secretin, sermorelin, superoxide dismutase (SOD), somatropins (growth hormone), somatostatin, streptokinase, sucrase, tetanus toxin fragment, tilactase, thrombins, thymosin, thyroid stimulating hormone, thyrotropin, tumor necrosis factor (TNF), TNF receptor-IgG Fc, tissue plasminogen activator (tPA), TSH, urate oxidase, urokinase, vaccines, and plant protein such as lectin and ricin.

6. The molecule of claim 4, wherein the protein is a protein prepared by recombinant DNA technology.

7. The molecule of claim 4, wherein the protein is selected from the group of proteins consisting of antibody fragments, single chain binding proteins, catalytic antibodies and fusion proteins.

8. The molecule of claim 4, wherein the protein is selected from the group of proteins consisting of antibodies, calcitonin, G-CSF, GM-CSF, erythropoietins, hemoglobins, interleukins, insulins, interferons, SOD, somatropin, TNF, TNF-receptor-IgC Fc.

9. The molecule of claim 3, wherein the small molecule biologically active agents are selected from the group of agents consisting of central nervous system-active agents, anti-infective, anti-neoplastic, antibacterial, anti-fungal, analgesic, contraceptive, anti-inflammatory, steroidal, vasodilating, vasoconstricting, and cardiovascular agents with at least one primary or secondary amino group.

10. The molecule of claim 3, wherein the small molecule biologically active agents are selected from the group of compounds consisting of daunorubicin, doxorubicin, idarubicin, mitoxantron, aminoglutethimide, amantadine, diaphenylsulfon, ethambutol, sulfadiazin, sulfamerazin, sulfamethoxazol, sulfalen, clinafloxacin, moxifloxacin, ciprofloxaxin, enoxacin, norfloxacin, neomycin B, sprectinomycin, kanamycin A, meropenem, dopamin, dobutamin, lisinopril, serotonin, acivicin and carbutamid.

11. The molecule of claim 1 or 2, wherein R4 is selected from the group of small substituents consisting of hydrogen, methyl, ethyl, ethoxy, methoxy, and other linear alkyls, cycloalkyls or branched alkyls and heteroalkyl comprising one to six carbon atoms.

12. The molecule of claim 1 or 2, wherein R1 is selected from the group of polymers consisting of polyalkyloxy-based polymers like poly(propylene glycol) or poly(ethylene glycol), dextran, chitosan, hyaluronic acid and derivatives, alginate, xylan, mannan, carrageenan, agarose, cellulose, starch, hydroxyethyl starch (HES) and other carbohydrate-based polmers, poly(vinyl alcohols), poly(oxazolines), poly(anhydrides), poly(ortho esters), poly(carbonates), poly(urethanes), poly(acrylic acids), poly(acrylamides) such as poly(hydroxypropylmethacrylamide) (HMPA), poly(acrylates), poly(methacrylates) like poly(hydroxyethylmethacrylate), poly(organophosphazenes), poly(siloxanes), poly(vinylpyrrolidone), poly(cyanoacrylates), poly(esters) such as poly(lactic acid) or poly(glycolic acids), poly(iminocarbonates), poly(amino acids) such as poly(glutamic acid), collagen, gelatin, copolymers, grafted copolymers, cross-linked polymers, and block copolymers from the above listed polymers.

13. The molecule of claim 1 or 2, wherein R1 is a hydrogel.

14. The molecule of claim 1 or 2, wherein R1 is a branched or hyperbranched polymer.

15. The molecule of claim 1 or 2, wherein R1 is a dendrimer or dense star polymer.

16. The molecule of claim 1 or 2, wherein R1 is a biopolymer.

17. The molecule of claim 16, wherein R1 is a protein.

18. The molecule of claim 17, wherein the protein is albumin, an antibody, fibrin, casein or any other plasma protein.

19. The molecule of any one of the above claims, wherein R1 further includes one or more biologically active substances.

20. The molecule of any one of the above claims, wherein the polymer of R1 has at least one functional group for linkage to X.

21. The molecule of claim 20, wherein the at least one functional group is selected from the group of functional groups consisting of carboxylic acid and activated derivatives, amino, maleimide, thiol, sulfonic acid and derivatives, carbonate and derivatives, carbamate and derivatives, hydroxyl , aldehyde, ketone , hydrazine , isocyanate, isothiocyanate, phosphoric acid and derivatives, phosphonic acid and derivatives, haloacetyl, alkyl halides, acryloyl, arylating agents like aryl fluorides, hydroxylamine, disulfides like pyridyl disulfide, vinyl sulfone, vinyl ketone, diazoalkanes, diazoacetyl compounds, epoxide, oxirane, and aziridine.

22. The molecular of claim 20 or 21, wherein the at least one functional group is selected from the group of functional groups consisting of thiol, maleimide, amino, carboxylic acid and derivatives, carbonate and derivatives, carbamate and derivatives, aldehyde, and haloacetyl.

23. The molecule of one of claims 20 to 22, wherein the bond or group formed between X and R1 is selected from the group of bonds or groups consisting of disulfide, S-succinimido, amide, amino, carboxylic ester, sulphonamide, carbamate, carbonate, there, oxime, hydrazone, urea, thiourea, phosphate, phosphonate.

24. The molecule of any one of claims 20 to 23, wherein the bonds or groups formed between X and R1 is selected from the group of bonds or groups consisting of S-succinimido, amide, carbamate, and urea.

25. The molecule of any one of the above claims, wherein A is selected from the group of leaving groups consisting of chloride, bromide, fluoride, nitrophenoxy, imidazolyl, N-hydroxysuccinimidyl, N-hydroxybenzotriazolyl, N-hydroxyazobenzotriazolyl, pentafluorphenoxy and N-hyroxysulfosuccinimidyl.

26. The molecule of claim 1 or 2, wherein the moiety 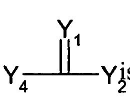 preferably selected from the group of moieties consisting of 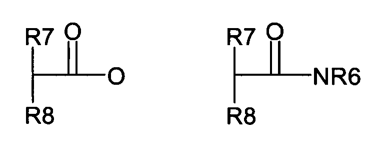 and

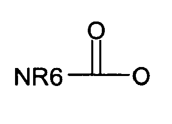

27. The molecule of claim 26, wherein the moiety 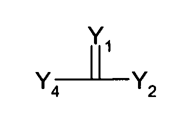 is

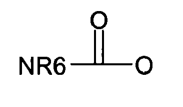

28. The molecule of claim 26 or 27 wherein R6 may be Nu-W.

29. The molecule of any one of claims 26 to 28 wherein Nu-W is 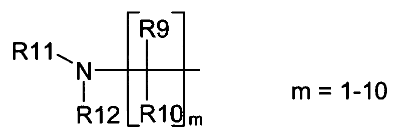 in which R9, R10, R11 and R12 are selected independently from hydrogen, substituted or non-substituted alkyl or heteroalkyl, substituted or non-substituted aryl or heteroaryl.

30. The molecule of claim 29, wherein R9, 10, R11 and R12 are selected independently from hydrogen or substituted or non-substituted alkyl.

31. The molecule of any of the above claims, wherein Nu is selected from the group of nucleophiles consisting of primary, secondary and tertiary amino groups, thiol, carboxylic acid, hydroxylamine and hydrazine.

32. The molecule of claim 25 or 26, wherein R7 and/or R8 are not hydrogen.

33. The molecule of claim 1 or 2, wherein Ar is selected from the group of moieties having the following structure: 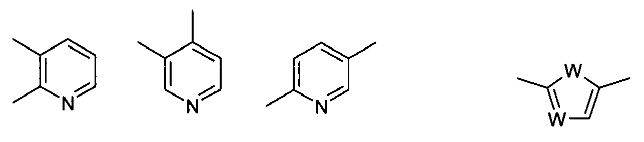 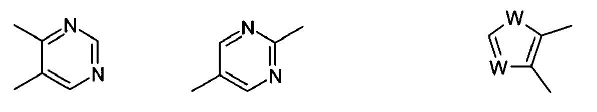 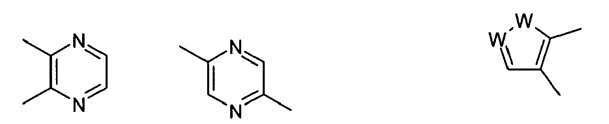 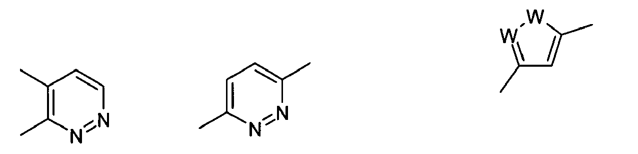 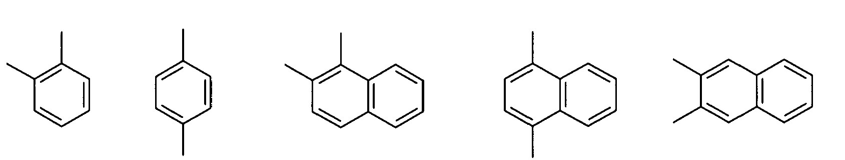 and wherein W is O, S, or N, independent from each other.

34. The molecule of claim 1 or 2 wherein Ar is a monocyclic or dicyclic aromatic hydrocarbon or aromatic heterocycle.

35. The molecule of claim 1 or 2, wherein the Ar is a five-membered or six-membered aromatic hydrocarbon or aromatic heterocycle.

36. Use of the molecule of any one of the above claims 1 to 35, in which T is a leaving group A, for covalent conjugation with a biologically active moiety and linkage with a carrier.

37. The use of claim 36, wherein the biologically active moiety is an amine-containing molecule.

38. The use of claim 36 or 37, wherein the biologically active moiety is a peptide, polypeptide or protein.

39. Method for the synthesis of a polymeric prodrug comprising:
- providing a starting molecule of Formula II 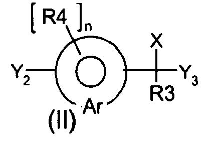
- synthesising at least one intermediate compound from the starting molecule of Formula II; and
- attaching an amine-containing biologically active moiety D to the at least one intermediate compound to form the polymeric prodrug;
wherein
Y₂ is selected from O, S, or NR6
Y₃ is selected from O or S
X is a spacer moiety such as R5-Y₆;
R3 is selected independently from hydrogen, substituted or non-substituted linear, branched or cyclical alkyl or heteroalkyl, aryl, substituted aryl, substituted or non-substituted heteroaryl, cyano, nitro, halogen, carboxy, carboxyalkyl, alkylcarbonyl or carboxamidoalkyl;
R4 is selected from hydrogen, substituted or non-substituted linear, branched or cyclical alkyl or heteroalkyl, aryl, substituted aryl, substituted or non-substituted heteroaryl, substituted or non-substituted linear, branched, or cyclical alkoxy, substituted or non-substituted linear, branched, or cyclical heteroalkyloxy, aryloxy or heteroaryloxy;
R5 is selected from substituted or non-substituted linear, branched or cyclical alkyl or heteroalkyl, aryl, substituted aryl, substituted or non-substituted heteroaryl, carboxyalkyl, alkylcarbonyl, carboxamidoalkyl;
R6 is selected from hydrogen, substituted or non-substituted linear, branched or cyclical alkyl or heteroalkyl, aryl, substituted aryl, substituted or non-substituted heteroaryl;
Y₆ is O, S, NR6, succinimide, maleimide, unsaturated carbon-carbon bonds or a heteratom containing a free electron pair;
n is zero or a positive integer and
Ar is a multi-substituted aromatic hydrocarbon or a multi-substituted aromatic heterocycle.

40. The method of claim 39, wherein a first one of the at least one intermediate compounds is an intermediate molecule of Formula III 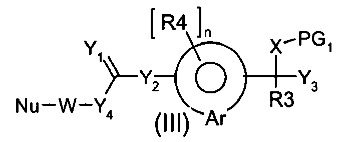 synthesised by acylating Y₂ with 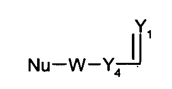 and attaching a first protecting group PG₁ wherein Nu is a nucleotide;
W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl; Y₁ is selected from the group of moieties consisting of O, S, or NR6;
Y₄ is selected from the group of moieties consisting of, or -C(R7)(R8)- O, NR6;
R7 and R8 are selected independently from hydrogen, substituted or non-substituted linear, branched or cyclical alkyl or heteroalkyl, aryl, substituted aryl, substituted or non-substituted heteroaryl, carboxyalkyl, alkylcarbonyl, carboxamidoalkyl, cyano or halogen.
PG₁ is a first protecting group or absent.

41. The method of claim 40, wherein a second one of the at least one intermediate compound is an intermediate compound of formula IV 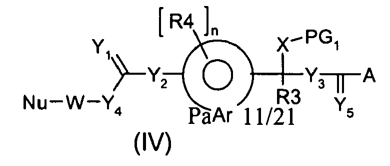 formed by activating the compound of Formula III with an activating agent, wherein A is a leaving group and Y₅ is selected from O or S.

42. The method of claim 41, wherein A is selected from chloride, bromide, fluoride, nitrophenoxy, imidazolyl, N-hydroxysuccinimidyl, N-hydroxybenzotriazolyl, N-hydroxyazobenzotriazolyl, pentafluorphenoxy and N-hydroxysulfosuccinimidyl.

43. The method of one of claims 41 or 42, wherein the activating agent is selected from the group of activating agents consisting of 4-nitrophenyl chloroformate or disuccinyl carbonate.

44. The method of one of claims 41 or 42, wherein the amine-containing biologically active moiety D is attached to the second one of the at least one intermediary compound (Formula IV) by displacement of the leaving group A.

45. The method of claim 44 comprising a step of removal of the reversible first protecting group PG₁ from the second one of the at least one intermediate compound.

46. The method of claim 45, wherein the step of removal of the reversible protection group PG₁ is carried out using a reagent selected from the group of reagents consisting of trifluoroacetic acid or DTT.

47. The method of any one of claims 45 or 46, further comprising a step of attaching a polymer R1 to X.

48. The method of claim 40, further comprising a step of removing the first protection group PG₁ and attaching a polymer R1 to X to form a third one of the at least one intermediate compound and having a Formula VI.

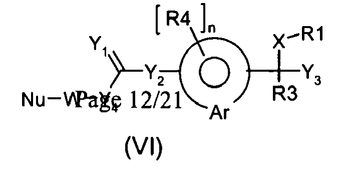

49. The method of claim 48, further comprising a step of activating the third one of the at least one intermediate compound by using an activating agent to form a fourth one of the at least one intermediate compound and having a Formula VII and wherein A is a leaving group

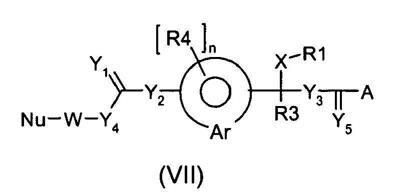

50. The method of claim 49, wherein A is selected from chloride, bromide, fluoride, nitrophenoxy, imidazolyl, N-hydroxysuccinimidyl, N-hydroxybenzotriazolyl, N-hydroxyazobenzotriazolyl, pentafluorphenoxy and N-hydroxysulfosuccinimidyl.

51. The method of claim 49 or 50, wherein the activating agent is selected from the group of activating agents consisting of 4-nitrophenyl chloroformate or disuccinyl carbonate.

52. The method of one of claim 49 to 51, wherein the amine-containing biologically active moiety D is attached to the compound of Formula VII by displacement of the leaving group A.

53. The method of claim 39, further comprising a step of reacting the starting molecule of Formula II with a first polymer R1 to attach the first polymer R1 to X.

54. The method of claim 53, further comprising a step of protecting Y₂ with a second protective group PG₂.

55. The method of claim 53 or 54, further comprising the step of activating the molecule with an activating agent and reacting with an amine-containing biologically active moiety D to form a fifth one of the at least one intermediate compound and having Formula X

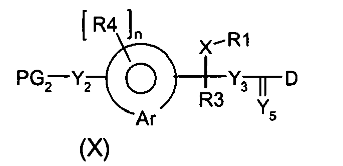

56. The method of claim 55, wherein the activating agent is selected from the group of activating agents consisting of 4-nitrophenyl chloroformate or disuccinyl carbonate

57. The method of claim 55 or 56, wherein the second protecting group PG₂ is removed from Y₂ and Y₂ is acylated with 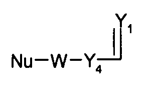 wherein W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl;
Nu is a nucleotide;
Y₁ is selected from the group of moieties consisting of O, S, or NR6;
Y₄ is selected from the group of moieties consisting of -C(R7)(R8)-, O, NR6; and
R7 and R8 are selected from hydrogen, substituted or non-substituted linear, branched or cyclical alkyl or heteroalkyl, aryl, substituted aryl, substituted or non-substituted heteroaryl, carboxyalkyl, alkylcarbonyl, carboxamidoalkyl, cyano or halogen.

58. The method of claim 57, wherein the removal of the second reversible group PG₂ is carried out using a reagent selected from the group of reagents consisting of trifluoroacetic acid or DTT.

59. The method of claim 39 comprising a step of attaching a removable first protecting group PG₁ to X and a removable second protecting group PG₂ to the starting compound of Formula II and thereafter activation using an activating agent to form a sixth one of the at least one intermediate compound and having Formula VIII, wherein A is a leaving group

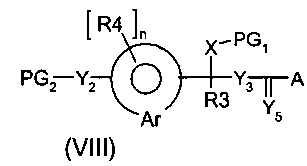

60. The method of claim 59, wherein A is selected from the group of leaving groups selected from the group of leaving groups consisting of chloride, nitrophenoxy, imidazolyly, N-hydroxysuccinimidyl, N-hydroxybenzotriazolyl, N-hydroxyazobenzotriazolyl, pentaflourphenoxy and N-hydroxysulfosuccinimidyl.

61. The method of claim 59 or 60, wherein the activating agent is selected from the group of activating agents consisting of 4-nitrophenyl chloroformate or disuccinyl carbonate.

62. The method of any one of claims 59 to 61, wherein the amine-containing biologically active moiety D is attached to the sixth one of the at least one intermediate compound by displacement of the leaving group A.

63. The method of claim 62 comprising a step of removing the first protecting group PG₁ from X and attaching a polymer R1.

64. The method of claim 63, wherein the second protecting group PG₂ is removed from Y₂ and Y₂ is acylated with 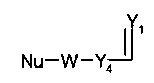 wherein W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl; Nu is a nucleotide;
Y1 is selected from the group of moieties consisting of O, S, or NR6;
Y4 is selected from the group of moieties consisting of -C(R7)(R8)-; O, or NR6; and
R7 and R8 are selected from hydrogen, substituted or non-substituted linear, branched or cyclical alkyl or heteroalkyl, aryl, substituted aryl, substituted or non-substituted heteroaryl, carboxyalkyl, alkylcarbonyl, carboxamidoalkyl, cyano or halogen.

65. The method of claim 62, wherein the second protecting group PG₂ is removed from Y₂ and Y₂ is acylated with 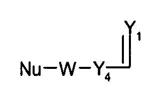 wherein W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl;
Nu is a nucleotide;
Y 1 is selected from the group of moieties consisting of O, S, or NR6;
Y4 is selected from the group of moieties consisting of -C(R7)(R8)- O, or NR6; and
R7 and R8 are selected from hydrogen, substituted or non-substituted linear alkyl, branched alkyl or cyclical alkyl, aryl, substituted aryl, substituted or non-substituted heteroalkyl or heteroaryl, carboxyalkyl, alkylcarbonyl, carboxamidoalkyl, cyano or halogen.

66. The method of claim 65 comprising a step of removing the first protecting group PG₁ from X and attaching a polymer R1.

67. The method of claim 43 comprising a step acylating Y₂ with 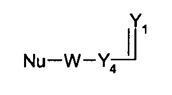 to form a third one of the at least one intermediate compound;
wherein W is selected from substituted or non-substituted linear, branched or cyclical alkyl, aryls, substituted aryls, substituted or non-substituted linear, branched or cyclical heteroalkyl, substituted or nonsubstituted heteroaryls, substituted or non-substituted linear, branched, or cyclical alkoxy, substituted or non-substituted linear, branched, or cyclical heteroalkyloxy, aryloxy, heteroaryloxycyano, carboxy, carboxyalkyl, alkylcarbonyl, carboxamidoalkyl;
Nu is a nucleotide;
Y₁ is selected from the group of moieties consisting of O, S, or NR6;
Y₄ is selected from the group of moieties consisting of -C(R7) (R8)-O or NR6; and
R7 and R8 are selected from hydrogen, substituted or non-substituted linear alkyl, branched alkyl or cyclical alkyl, aryl, substituted aryl, substituted or non-substituted heteroalkyl or heteroaryl, carboxyalkyl, alkylcarbonyl, carboxamidoalkyl, cyano or halogen.

68. The method of claim 67, further comprising a step of activating the third one of the at least one intermediate compound by using an activating agent to form a seventh one of the at least one intermediate compound and having a Formula VII and wherein A is a leaving group

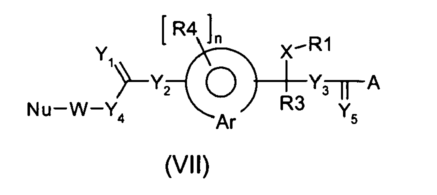

69. The method of claim 68, wherein A is selected from the group of leaving groups consisting of chloride, nitrophenoxy, imidazolyl, N-hydroxysuccinimidyl, N-hydroxybenzotriazolyl, N-hydroxyazobenzotriazolyl, pentafluorphenoxy and N-hyroxysulfosuccinimidyl.

70. The method of claim 68 or 69, wherein the activating agent is selected from the group of activating agents consisting of 4-nitrophenyl chloroformate or disuccinyl carbonate.

71. The method of one of claims 68 to 70, wherein the amine-containing biologically active moiety D is attached to the compound of Formula VII by displacement of the leaving group A.

72. The method of any one of claims 39 to 71 wherein the starting molecule of Formula II is replaced by a starting molecule of Formula IIb 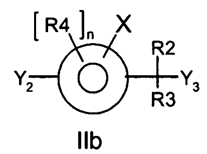 wherein R2 is selected from hydrogen, substituted or non-substituted linear, branched or cyclical alkyls or heteroalkys, aryls, substituted or non-substituted heteroaryls, cyano, nitro, halogen, carboxy, carboxyalkyl, alkylcarbonyl or carboxamidoalkyl.

73. A method for hydrolysing the molecule of any one of claims 1 to 35 comprising a step of placing the molecule in solution with a pH of approximately 7.4

74. The method of claim 73, wherein the solution is an extra-cellular fluid.

75. Method of administration of an amine-containing moiety to a living organism comprising:
- a first step of attaching the amine-containing moiety through an amino group to a carrier molecule by means of a nucleophile-containing linker;
- a second step of administering the amine-containing moiety and the carrier molecule to the living organism; and
- a third step of cleaving the amine-containing moiety from the carrier molecule by means of a substantially non-enzymatic reaction.

76. The method of claim 75, wherein the amine-containing moiety is a biologically active moiety.

77. The method of claim 76 wherein the biologically active moiety is selected from the group of biologically moieties consisting of small molecule biologically active agents or biopolymers.

78. The molecule of claim 76, wherein the biopolymers are selected from the group of biopolymers consisting of proteins, polypeptides, oligonucleotides and peptide nucleic acids.

79. The method of claim 78, wherein the polypeptides are selected from the group of polypeptides consisting of ACTH, adenosine deaminase, agalsidase, albumin, alfa-1 antitrypsin (AAT), alfa-1 proteinase inhibitor (API), alteplase, anistreplase, ancrod serine protease, antibodies (monoclonal or polyclonal, and fragments or fusions), antithrombin III, antitrypsins, aprotinin, asparaginases, biphalin, bone-morphogenic proteins, calcitonin (salmon), collagenase, DNase, endorphins, enfuvirtide, enkephalins, erythropoietins, factor VIIa, factor VIII, factor VIIIa, factor IX, fibrinolysin, fusion proteins, follicle-stimulating hormones, granulocyte colony stimulating factor (G-CSF), galactosidase, glucagon, glucocerebrosidase, granulocyte macrophage colony stimulating factor (GM-CSF), phospholipase-activating protein (PLAP), gonadotropin chorionic (hCG), hemoglobins, hepatitis B vaccines, hirudin, hyaluronidases, idurnonidase, immune globulins, influenza vaccines, interleukins (1 alfa, 1 beta, 2, 3, 4, 6, 10, 11, 12), IL-1 receptor antagonist (rhIL-1ra), insulins, interferons (alfa 2a, alfa 2b, alfa 2c, beta 1a, beta 1b, gamma 1a, gamma 1b), keratinocyte growth factor (KGF), transforming growth factors, lactase, leuprolide, levothyroxine, luteinizing hormone, lyme vaccine, natriuretic peptide, pancrelipase, papain, parathyroid hormone, PDGF, pepsin, platelet activating factor acetylhydrolase (PAF-AH), prolactin, protein C, octreotide, secretin, sermorelin, superoxide dismutase (SOD), somatropins (growth hormone), somatostatin, streptokinase, sucrase, tetanus toxin fragment, tilactase, thrombins, thymosin, thyroid stimulating hormone, thyrotropin, tumor necrosis factor (TNF), TNF receptor-IgG Fc, tissue plasminogen activator (tPA), TSH, urate oxidase, urokinase, vaccines, and plant protein such as lectin and ricin.

80. The method of claim 78, wherein the protein is a protein prepared by recombinant DNA technology.

81. The method of claim 78, wherein the protein is selected from the group of proteins consisting of antibody fragments, single chain binding proteins, catalytic antibodies and fusion proteins.

82. The method of claim 77, wherein the small molecule biologically active agents are selected from the group of agents consisting of central nervous system-active agents, anti-infective, anti-neoplastic, antibacterial, anti-fungal, analgesic, contraceptive, anti-inflammatory, steroidal, vasodilating, vasoconstricting, and cardiovascular agents with at least one primary or secondary amino group.

83. The method of claim 77, wherein the small molecule biologically active agents are selected from the group of compounds consisting of daunorubicin, doxorubicin, idarubicin, mitoxantron, aminoglutethimide, amantadine, diaphenylsulfon, ethambutol, sulfadiazin, sulfamerazin, sulfamethoxazol, sulfalen, clinafloxacin, moxifloxacin, ciprofloxaxin, enoxacin, norfloxacin, neomycin B, sprectinomycin, kanamycin A, meropenem, dopamin, dobutamin, lisinopril, serotonin, acivicin, and carbutamid.

84. The method of one of claims 75 to 83, wherein the third step is carried out in an extra-cellular fluid.

85. The method of one of claims 75 to 84, wherein the substantially non-enzymatic reaction comprises a step of hydrolysis.

86. The method of one of claims 75 to 85, wherein the substantially non-enzymatic reaction comprises a step of intramolecular cyclization.

87. The method of one of claims 75 to 86, further comprising a step of sterically protecting at least part of the linker by a sterically demanding carrier.

88. In an amine-containing moiety attached to a carrier by a nucleophile-containing linker, a method of cleaving the amine-containing moiety from the carrier by a substantially non-enzymatic reaction of the nucleophile-containing linker.

89. The method of claim 88, wherein the substantially non-enzymatic reaction is carried out at a pH of approximately 7.4.

90. The method of claim 88 or 89, wherein the amine-containing moiety attached to the carrier is cleaved in an extra-cellular fluid.

91. The method of one of claims 88 to 90, wherein the substantially non-enzymatic reaction comprises a step of hydrolysis.

92. The method of one of claims 88 to 91, wherein the substantially non-enzymatic reaction comprises a step of intramolecular cyclization.

93. The method of one of claims 88 to 92, further comprising a step of sterically protecting at least part of the nucleophile-containing linker by a sterically demanding carrier.

94. The method of any one of claims 88 to 93, wherein the amine-containing moiety is a biologically active moiety.
